(19) Europäisches Patentamt
European Patent Office
Office européen des brevets

(11) **EP 4 076 238 B1**

(12) **EUROPEAN PATENT SPECIFICATION**

(45) Date of publication and mention
of the grant of the patent:
**29.11.2023 Bulletin 2023/48**

(51) International Patent Classification (IPC):
**A61B 18/04** (2006.01)     **A61B 18/18** (2006.01)
**A61B 18/00** (2006.01)

(21) Application number: **20838385.1**

(22) Date of filing: **15.12.2020**

(52) Cooperative Patent Classification (CPC):
**A61B 18/042; A61B 18/1815;** A61B 2018/00785;
A61B 2018/1853; A61B 2018/1861;
A61B 2217/005; A61B 2217/007

(86) International application number:
**PCT/EP2020/086163**

(87) International publication number:
**WO 2021/122557 (24.06.2021 Gazette 2021/25)**

(54) **ELECTROSURGICAL INSTRUMENT AND APPARATUS**

ELEKTROCHIRURGISCHES INSTRUMENT UND GERÄT

INSTRUMENT ET APPAREIL ÉLECTROCHIRURGICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2019 GB 201918615**

(43) Date of publication of application:
**26.10.2022 Bulletin 2022/43**

(73) Proprietor: **Creo Medical Limited
Chepstow, Monmouthshire NP16 5UH (GB)**

(72) Inventor: **HANCOCK, Christopher Paul
Chepstow, Monmoutshire NP16 5UH (GB)**

(74) Representative: **Mewburn Ellis LLP
Aurora Building
Counterslip
Bristol BS1 6BX (GB)**

(56) References cited:
WO-A2-2004/026150          GB-A- 2 487 199
JP-A- 2004 008 524          US-A1- 2001 034 519
US-A1- 2013 085 514          US-A1- 2013 253 498
US-A1- 2016 113 700          US-A1- 2017 014 184

## Description

### FIELD OF THE INVENTION

The invention relates to an electrosurgical apparatus in which radiofrequency and/or microwave frequency energy is used to sterilise biological tissue via non-thermal plasma, and to resurface biological tissue via thermal plasma. Additionally, the electrosurgical apparatus includes means for debriding biological tissue, for example, prior to sterilisation and/or resurfacing. Specific embodiments are also capable of resurfacing biological tissue using non-ionising radiation. Various embodiments may be suitable for use in open surgery, or for insertion down the instrument channel of a scoping device, such as, a laparoscope or an endoscope.

### BACKGROUND TO THE INVENTION

Argon plasma coagulation (APC) or argon beam coagulation (ABC) is a known surgical technique for controlling surface bleeding in a manner that does not require physical contact between a surgical probe delivering the plasma and the lesion. APC can be performed endoscopically, whereby a jet of argon gas is directed through a probe passed through an endoscope. Ionization of the argon gas as it is emitted creates the plasma that causes coagulation.

To strike plasma it is desirable to have a high electric field (e.g. by directly applying a high voltage or setting up a high impedance condition that causes a high voltage to exist). Typically this is done by applying a high RF voltage pulse (e.g. 500 V to 2 kV) between an active electrode and a return electrode that are separated by a small distance, e.g. less than 1 mm, for a short duration of time, e.g. in a range from 1 ms to 10 ms. The high electric field can break down the gas to initiate a plasma. In one embodiment discussed in WO 2009/060213, a high voltage (high impedance) condition is set up using a flyback circuit that uses a low frequency (e.g. radiofrequency) oscillator circuit e.g. running at 100 kHz and a transformer whose primary winding is connected to the low voltage oscillator circuit by a suitable driver and switching device (e.g. gate drive chip and a power MOSFET or BJT). The arrangement generates high voltage pulses or spikes which strike or otherwise initiate the plasma. Once struck, the impedance drops and the plasma may be maintained by a supply of microwave energy.

Sterilisation is an act or process that destroys or eliminates all form of life, especially micro-organisms. During the process of plasma sterilisation, active agents are produced. These active agents are high intensity ultraviolet photons and free radicals, which are atoms or assemblies of atoms with chemically unpaired electrons. An attractive feature of plasma sterilisation is that it is possible to achieve sterilisation at relatively low temperatures, such as body temperature. Plasma sterilisation also has the benefit that it is safe to the operator and the patient.

Tissue resurfacing or reepithelialisation is the process by which the skin and mucous membranes replace superficial epithelial cells damaged or lost in a wound. Epithelial cells at the edge of a wound proliferate almost immediately after injury to cover the denuded area. Epithelialization is an essential component of wound healing and is used as a defining parameter of a successful wound closure. A wound cannot be considered healed in the absence of re-epithelialization.

Debridement is the medical removal of dead, damaged, or infected tissue to improve the healing potential of the remaining healthy tissue. Removal may be surgical, mechanical, chemical, and autolytic (self-digestion). Debridement is an important part of the healing process for burns and other serious wounds.

There is a continuing need to improve instruments and apparatuses for treating wounds and for reducing wound healing time. There is also a continuing need to improve instruments and apparatuses for debriding, sterilizing and resurfacing of biological tissue. Document US2016113700A1 relates to an electrosurgical device that is capable of both generating a plasma to perform surface coagulation and emitting a non-ionizing microwave field (in the absence of plasma) to perform coagulation at a deeper level.

### SUMMARY OF THE INVENTION

The invention is defined in the appended independent claim. Further embodiments are defined in appended dependent claims. At its most general, the present invention provides an electrosurgical instrument that includes a means of delivering non-thermal plasma and thermal plasma on to biological tissue. Also, the instrument includes means for debriding biological tissue. As such, the electrosurgical instrument may be a wound treatment apparatus or device for debriding, sterilizing and resurfacing a wound. Additionally or alternatively, the electrosurgical instrument may find utility in other treatment (e.g. debridement, sterilisation, and/or reepithelialisation) applications, for example, treating bodily infections, such as, ear infections (e.g. otitis media) or urinary tract infections. Further, the electrosurgical instrument may find utility in treating (e.g. debriding, sterilizing and/or resurfacing) bodily treatment sites for receiving medical implants, such as metal implants, and for debriding and/or sterilizing such implants.

The means for debriding may include one or more of: an adhesive region for scraping or scrubbing biological tissue; a deployable debriding tool for scraping or scrubbing biological tissue; and, a liquid passage for injecting/extracting liquid to/from biological tissue. This functionality may be useful for removing dead, damaged or infected tissue from a wound, and for removing foreign objects (e.g. dirt, grit, contaminants) from the wound. Such functionality can assist in wound healing. Additionally, this functionality may be useful in removing infected tissue and/or mucus from infection sites, for example, in the ear or urinary tract. Further, this functionality may be useful in removing infected tissue from a bodily treatment site for receiving a medical implant, and for removing infected tissue from the implant itself.

The non-thermal plasma functionality may be useful for sterilising biological tissue. For example, after debriding a wound, the non-thermal plasma may be used to kill or significantly reduce bacteria in the wound. Such functionality can assist in wound healing. Additionally, this functionality may be useful in removing or reducing bacterial from bodily infection sites, such as, infection sites in the ear or urinary tract. Further, this functionality may be useful in removing or reducing bacteria in bodily implant sites, such as those for receiving medical implants, and for removing or reducing bacteria on the implant itself.

The thermal plasma functionality may be useful for resurfacing (aka reepithelializing) biological tissue. For example, after debriding and then sterilizing a wound, the thermal plasma may be used to resurface the wound in order to close the wound bed and to promote wound healing.

Further, the instrument may include a means for delivering non-ionised microwave radiation to the biological tissue. This functionality may be used to resurface the biological tissue, to promote wound healing. For example, the non-ionising radiation may create a surface ablative effect which resurfaces tissue. The non-ionised microwave energy may be used as an alternative to thermal plasma. For example, after debriding and then sterilizing a wound, the non-ionised microwave energy may be used to resurface the wound in order to promote wound healing.

According to the invention, there is provided an electrosurgical instrument (e.g. wound treatment device) comprising: an elongate probe comprising a coaxial cable for conveying radiofrequency (RF) and/or microwave frequency electromagnetic (EM) energy, and a probe tip connected at the distal end of the coaxial cable for receiving the RF and/or microwave energy; a gas passage for conveying gas through the elongate probe to the probe tip; and a debriding apparatus for debriding biological tissue (e.g. in an area outward from the probe tip, i.e. at or near the distal end of the probe tip), wherein the coaxial cable comprises an inner conductor, an outer conductor and a dielectric material separating the inner conductor from the outer conductor, wherein the probe tip comprises a first electrode connected to the inner conductor of the coaxial cable and a second electrode connected to the outer conductor of the coaxial cable, and wherein the first electrode and the second electrode are arranged to produce an electric field from the received RF and/or microwave frequency EM energy across a flow path of gas received from the gas passage to produce a thermal or non-thermal plasma in an area outward from the probe tip (e.g. an area at or near the distal end of the probe tip). In use, the probe tip is connected to receive radiofrequency (RF) and/or microwave frequency energy from a generator, and also defines a flow path for a gas.

In a first configuration, the probe tip defines a bipolar (e.g. coaxial) structure to produce a high electric field from the received RF and/or microwave frequency energy across the flow path for the gas to strike and sustain plasma. For example, a short pulse (e.g. having a duration of 10 ms or less, e.g. between 1 ms and 10 ms) of RF energy may be used to strike the plasma. The outlet for the plasma is at the distal end of the probe tip, and is therefore delivered into the area outward from the probe tip.

The first electrode and the second electrode may be movable relative to each other into a second configuration in which the first electrode extends distally beyond the second electrode to form a radiating structure for emitting a microwave EM field outwardly from the probe tip. In the second configuration, the probe tip defines an antenna structure to emit non-ionising microwave energy into tissue. This non-ionising microwave energy can be used to resurface the tissue, for example, after the tissue has been debrided and/or sterilised. The antenna structure may be a radiating monopole antenna, which may take the form of a cylinder, a ball, a stiff wire or a helix or a turnstile antenna that is capable of emitting outwardly (i.e. away from the probe) an electric field from the received microwave frequency energy. Thus, in the first configuration the device may use one or both of RF energy and microwave energy, whereas in the second configuration, the device uses primarily microwave energy. The waveform of the RF energy used to strike the plasma may be a high amplitude pulse.

It may be possible for the non-ionising microwave field to be generated without relative movement of the first and second electrodes, e.g. simply by delivering microwave energy in the absence of gas. However, a more uniform field effect can be produced in the area encircled by the loop if the second electrode is set back from the first electrode, i.e. the first electrode protrudes distally from the second electrode.

The first electrode and the second electrode form active and return electrodes for an RF signal conveyed by the coaxial cable.

In the first configuration, the plasma may be struck using RF or microwave energy. Microwave energy may be used to sustain the plasma after it is struck. This arrangement may offer an advantage over RF plasma used in conventional electrosurgical systems, where the electric field may collapse due to the capacitance of the cable and loading caused by tissue variations.

The impedance of the plasma is preferably matched to the impedance of the applicator (and energy delivery system) at the frequency of the microwave energy to enable efficient transfer of the microwave energy, produced by the microwave source, into the plasma. Where microwave energy is used, the applicator and/or generator may be tuned (statically or dynamically) to ensure that the plasma is matched into the load presented by the tissue. At microwave frequencies, the cable forms a distributed element transmission line, where the impedance match between applicator and energy source is determined by the source impedance of the microwave generator, the characteristic impedance of the cable (transmission line), the impedance of the applicator structure itself and the impedance of the tissue. If the characteristic impedance of the cable is the same as the output impedance of the source then all of the microwave power will be delivered into the applicator, less the attenuation caused by the cable (dielectric and conductor losses). If the impedance of the applicator and the tissue is the same as the characteristic impedance of the cable, then the maximum power available at the source will be transferred into the plasma/tissue load. Adjustments may be made to applicator structure in order to maintain the best impedance match between the applicator and the plasma/tissue load, as explained below. Adjustments may also be made at the generator or at the interface between the distal end of the first cable and the proximal end of the second (instrument) cable. These adjustments may be in the form of a change of capacitance and/or inductance of a matching network, i.e. stub tuning.

The gas may be argon, or any other suitable gas, e.g. carbon dioxide, helium, nitrogen, a mixture of air and any one of these gases, i.e. 10% air/90% helium. The high electric field for striking the plasma may be caused by creating a high impedance condition for either the RF EM energy or the microwave EM energy at the probe tip. This can be achieved through the selection of a suitable geometry for the first and second electrodes. For example, a piece of insulating dielectric material, such as quartz or other similarly low loss material, may be located between the first and second electrodes in the first configuration. This reduces the electric field inside the insulating dielectric material and causes a consequent increase in the electric field in the gas-filled gap beside the insulation dielectric material. In the first configuration, the second electrode may be arranged to extend past (e.g. more distally than) the first conductor to ensure that non-ionising radiation is not emitted.

In a preferred embodiment, the instrument is capable of receiving both RF and microwave EM energy. The RF EM energy may be for striking the plasma, and may be received as a high voltage pulse. The microwave EM energy is for sustaining the plasma, i.e. delivering power into the plasma to maintain the state of ionisation. This may also be received as a pulse. The plasma may be struck repeatedly in a manner to produce a quasi-continuous beam of plasma. The advantage of this arrangement over conventional APC device which use only RF EM energy is that the plasma will not collapse due to capacitive loading or changing from a dry to wet environment. Moreover, the dual configuration nature of the instrument enables it to switch to a state suitable for tissue resurfacing using non-ionised microwave energy, where the second electrode (and the insulating dielectric material) are withdrawn to a distance where the first electrode is exposed such that is acts as a radiating microwave monopole antenna structure as discussed below.

In conventional RF plasma devices, the cable capacitance and high voltages demand a high RF drive current to maintain the plasma. For example, we can apply the well-known equation $I = C\frac{dV}{dt}$ to a quarter cycle at 400 kHz, where $dt$ is (2.5/4) $\mu$s = 625 ns. If the capacitance of the cable is 300 pF and the required peak voltage is 400 V, then I = 300 × 400/625 = 0.192 A, which is relatively high. The microwave signal has a much lower voltage, e.g. around 20 V, and therefore overcomes this disadvantage.

It may also be possible to strike the plasma using the microwave frequency energy, e.g. by using a microwave resonator or an impedance transformer, i.e. a quarter wave transformer that transforms a low voltage to a higher voltage to strike plasma using a higher impedance transmission line that is a quarter wave (or an odd multiple thereof) long at the frequency of operation. This high impedance line may be switched in to strike plasma and switched out (i.e. to return to a lower impedance line) once the plasma has been struck and it is required to sustain plasma. A power PIN or varactor diode may be preferably used to switch between the two states, although it may be possible to use a co-axial or waveguide switch.

The elongate probe may comprise a sleeve surrounding the coaxial cable. The sleeve may act to protect the coaxial cable, but may also define the gas passage, e.g. as a space between an inside surface of the sleeve and an outside surface of the coaxial cable. The gas passage may have an input port located at a proximal end of the sleeve for connecting to a source of gas (e.g. a pressurised gas canister or the like).

The sleeve may further be the means for causing relative movement between the first and second electrodes. Relative movement between the first and second electrodes may be achieved by sliding a conductive (e.g. metallic) catheter over a microwave co-axial cable, whose outer conductor may also metallic. In this configuration the inner surface of the catheter (or tube that slides over the co-axial cable) must make good electrical contact with the outer conductor of the coaxial cable. This may be achieved by providing a gas permeable conductive structure that is slidable relative to the second electrode or outer electrode of the coaxial cable and permits gas to flow through it. The gas permeable conductive structure may be any one of: a conductive mesh; a cage of radially extending conductive wires or springs; and a plurality of circumferentially spaced radially protruding dents. The gas permeable conductive structure may thus provide a plurality of (e.g. four or more) circumferential connections or point contacts will need to be made to ensure that a good electrical connection is made for the microwave signal. This solution may provide a balance between having enough connection points to create an appropriate environment for the microwave energy to propagate, to allow enough gas to flow and allow the outer catheter to be moved over the co-axial cable with relative ease.

In one embodiment, the second electrode may be mounted on or formed at the distal end of the sleeve, and the sleeve may be retractable relative to the coaxial cable. In other words, the sleeve may be capable of being drawn back to reveal the first electrode at the probe tip. The sleeve may be coaxial with the coaxial cable. The first and second electrodes may thus be coaxial with each other in the first configuration. The second electrode may be an annular band of conductive material on the distal end of the sleeve. The dielectric material mentioned above may be a quartz collar mounted on the sleeve inwardly of the annular band. Alternatively or additionally, the dielectric material may be part of the inner electrode, as discussed below.

The retracting sleeve may comprise two or more telescoping sections. The telescoping sections may have a fluid tight seal therebetween to prevent the gas from escaping. The slidable outer sleeve may be retracted or extended using a mechanical or electromechanical system, i.e. a mechanical slider, a linear motor or a stepper motor arrangement. As explained below, the position of the outer sleeve with respect to the outer conductor of the co-axial cable may be determined by a return loss or impedance match/mismatch measurement made using a reflected power or forward and reflected power measurement, i.e. a reflectometer or VSWR bridge measurement, using a detector(s) within the generator or within the probe.

The first electrode may be a radiating microwave monopole antenna structure coupled to receive RF and/or microwave EM energy from the coaxial cable. The outer conductor of the coaxial cable may be grounded to form an unbalanced feed or may be floating to form a balanced feed to the antenna, i.e. where the voltage on both conductors is going up and down. Preferably the first electrode is shaped to act as a microwave antenna for emitting a microwave field corresponding to the received microwave EM radiation. For example, the monopolar radiating structure may comprise a cylinder of dielectric material having a hemispherical distal end surrounding a length of the inner conductor of the coaxial cable which protrudes beyond the outer conductor and extends through the cylinder of dielectric material to protrude at its hemispherical distal end. Other distal end shapes are possible, e.g. ball or flat end. The cylinder may be made of low loss ceramic material. The presence of the dielectric cylinder can improve the energy delivery into tissue, e.g. by reducing the amount of reflected power. The end of the length of inner conductor that protrudes from the hemispherical distal end of the cylinder may be rounded, e.g. shaped into a hemisphere, to provide a more uniform emitted field.

The probe may be used in open surgery (e.g. be handheld) or laparoscopically or may be dimensioned to be insertable through a scoping device, e.g. through the instrument channel of an endoscope, gastroscope, bronchoscope or the like. For example, the coaxial cable may have a diameter of 2.5 mm or less, preferably 2.2 mm or less. The sleeve may have an outer diameter less than 2.6 mm, preferably less than 2.5 mm. For larger laparoscopic instruments, the outer diameter may be 3 mm or more, and larger diameter co-axial cable may be used. In an embodiment, the probe may be about 30cm long.

The debriding apparatus may include an abrasive region on a distal outer surface of the probe tip of the instrument for debriding biological tissue in the area outward from the probe tip (i.e. at or near the probe tip). For example, an operator may manipulate the position and/or orientation of the probe tip to bring the abrasive region into contact with biological tissue which requires debriding, for example, a wound or other treatment site (e.g. bodily infection site or bodily implant site). The abrasive region can be used to scrape or scratch the surface of the biological tissue to debride. The abrasive region may be a coating which is applied directly to an outer surface of the instrument. Alternatively, the abrasive region may be a patch which is manufactured separately and then bonded to the instrument (e.g. via an adhesive or a mechanical fixing). The abrasive region may be any shape, for example, square, rectangular, circular, oval, regular or irregular. Also, the abrasive region may cover the whole circumference of the instrument and so be substantially annular or ring shaped. Alternatively, the abrasive region may cover only a portion of the circumference. The abrasive region may be on a distal end face of the probe tip. The abrasive region may include abrasive elements with sharp points or edges for gripping and removing biological material and foreign objects to clean a wound or other treatment site (e.g. bodily infection site or bodily implant site).

Additionally or alternatively, the debriding apparatus may include a deployable debriding tool, such as, for example, a brush or pad. In an embodiment, the probe tip includes a holder (e.g. recess or cavity) for receiving the debriding tool, and the debriding tool is moveable between a stowed position, in which the debriding tool is enclosed (e.g. contained, housed) within the holder, and a deployed position, in which the debriding tool protrudes into the area outward from the probe tip for debriding biological tissue. In this case, the sleeve may comprise a rotatable braided cable to permit adjustment of an orientation of the debriding apparatus. An opening of the holder may be on a distal end face of the elongate probe so that the debriding tool deploys directly into the area outward of the probe tip, i.e. the area into which the plasma (thermal or non-thermal) or non-ionising radiation is delivered. The debriding tool and holder may be housed within a guide structure which is bonded to an outer surface of the elongate probe. Alternatively, the holder and debriding tool may be integrated into the internal structure of the elongate probe. In an embodiment, the electrosurgical instrument includes a deployment mechanism for moving the debriding tool between the stowed and deployed positions. The deployment mechanism may comprise an actuator, e.g. lever, pull wire or pull arm, located at the proximal end of the probe, e.g. a sliding or rotating mechanism that is moved by hand (e.g. via a handle). However, it is also contemplated herein to control the movement of the deployable debriding tool in an automated manner, e.g. using an electromechanical mechanism (e.g. including a linear motor, a stepper motor, a piezoelectric actuator, and a magnetostrictive actuator). For example, in one embodiment, there may be a controller arranged to automatically move the debriding tool.

Additionally or alternatively, the debriding apparatus may include a liquid passage for conveying liquid through the elongate probe and into or out of the area outward of the probe tip for debriding biological tissue. The liquid injected or supplied into the treatment site may be water, saline or some other liquid suitable for debriding, e.g. for cleaning/irrigating a treatment site (e.g. wound, infection site, implant site) by dislodging: dead, damaged or infected tissue, or foreign objects such as grit, dirt and other contaminants which may hinder healing. As such, liquid extracted from the treatment site includes solids such as: dead, damaged or infected tissue, or foreign objects such as grit, dirt and other contaminants. In one embodiment, the elongate probe comprises a jacket surrounding the sleeve, and the liquid passage is a space between the inside surface of the jacket and an outside surface of the sleeve. The jacket may be structurally similar to the sleeve, that is, the sleeve may provide an inner sleeve and the jacket may provide an outer sleeve. The liquid passage may have an input port located at a proximal end of the jacket for connecting to a source of liquid (e.g. a tank or container or the like). The jacket may have a similar construction to the sleeve mentioned above, in order that the first electrode is movable relative to the second electrode. That is, the jacket and sleeve may move together as the first electrode is moved relative to the second electrode. Specifically, the jacket may be coaxial with the coaxial cable and the jacket may be retractable relative to the coaxial cable. The retracting jacket may comprise two or more telescoping sections. The telescoping sections may have a fluid tight seal therebetween to prevent the liquid from escaping. The slidable outer jacket may be retracted or extended using a mechanical or electromechanical system, i.e. a mechanical slider, a linear motor or a stepper motor arrangement.

In another embodiment in which the jacket is absent, the space between the inside surface of the sleeve and the outside surface of the coaxial cable is divided or partitioned into the gas passage and the liquid passage. For example, one or more dividing structures or elements may be present inside the space to partition it into the gas and liquid passages, such that fluid cannot transfer from the gas passage to the liquid passage or vice versa. In this case, the gas and liquid passages may be connected to respective ports in the proximal end of the probe. Such a configuration may be advantageous where there is a need to keep the outside profile of the probe as small (e.g. thin) as possible, for example, where the probe is to be inserted down the instrument channel of a scoping device. It is to be understood that where the jacket is present, the jacket may define an outer profile of the instrument. However, when the jacket is not present, the sleeve may define the outer profile of the instrument.

Additionally, the liquid passage may be divided or partitioned into two or more channels. Of these channels, at least some (aka first channels) are for conveying liquid from a proximal end of the elongate probe to a distal end of the elongate probe, out of the distal end of the probe tip, and into a treatment site in the area outward of the probe tip. Also, at least some of the channels (aka second channels) are for conveying liquid and solids (e.g. biological material or foreign objects) from the treatment site in the area outward of the probe tip, into the distal end of the probe tip and through the elongate probe from its distal end to its proximal end.

The invention may also be expressed as an electrosurgical apparatus comprising: a radiofrequency (RF) signal generator for generating RF electromagnetic (EM) radiation having a first frequency; a microwave signal generator for generating microwave EM radiation having a second frequency that is higher than the first frequency; an electrosurgical instrument as described above connected to receive the RF EM radiation and the microwave EM radiation; a feed structure for conveying the RF EM radiation and the microwave EM radiation to the elongate probe, the feed structure comprising an RF channel for connecting the elongate probe to the RF signal generator, and a microwave channel for connecting the elongate probe to the microwave signal generator, a gas feed connected to supply gas to the electrosurgical instrument, wherein the apparatus is operable to debride biological tissue in the area outward from the probe tip (e.g. at or near the distal end of the probe tip), and wherein the apparatus is operable to deliver a thermal or non-thermal plasma in the area outward from the probe tip (e.g. to sterilize tissue (via non-thermal plasma) or to resurface tissue (via thermal plasma)).

The first electrode and the second electrode may be movable relative to each other into a second configuration in which the first electrode extends distally beyond the second electrode to form a radiating structure for emitting a microwave EM field outwardly from the probe tip, wherein the apparatus is operable to emit a non-ionising electric field outwardly from the probe tip when the first electrode and the second electrode are in the second configuration without gas supplied to thereto.

The apparatus may comprise a strike signal generation circuit arranged to cause a pulse (or pulses) of RF EM radiation to be delivered to the probe to generate the high electric field across the flow path for striking the plasma, wherein the strike signal generation circuit includes control circuitry arranged to use a detectable characteristic of a pulse of microwave EM radiation on the microwave channel to trigger generation of the pulse of RF EM radiation. The RF EM radiation is thus used to strike the plasma, whereas the microwave EM radiation is used to sustain the plasma. By coordinating the delivery of an RF strike pulse with a pulse of microwave EM radiation as described above, the apparatus is capable of striking the plasma with greater certainty.

The apparatus may further comprise a microwave signal detector for sampling forward and reflected power on the microwave channel and generating therefrom a microwave detection signal indicative of the microwave power delivered by the probe; and a controller in communication with the microwave signal detector to receive the microwave detection signal, wherein the controller is operable to select a first energy delivery profile for the microwave EM radiation, the first energy delivery profile for the microwave EM radiation being for sterilisation of tissue (via non-thermal plasma generation), wherein the controller comprises a digital microprocessor programmed to output a first microwave control signal for the microwave signal generator, the first microwave control signal being for setting the first energy delivery profile for the microwave EM radiation, and wherein the controller is arranged to determine a state for the first microwave control signal based on the received microwave detection signal. The arrangement may be used to measure the reflected microwave signal, whereby the microwave detection signal is representative of whether or not a plasma has been struck. The signal detector may also be arranged to continuously monitor the forward and reflected microwave EM radiation to ensure that the best impedance match is maintained during plasma delivery. The microwave signal detector may comprise forward and reflected signal detectors (e.g. suitable directional power couplers on the microwave channel). The detectors may be arranged to detect signal magnitude only, e.g. they may be diode detectors. Alternatively, the detectors may be arranged to detect magnitude and phase, e.g. they may be heterodyne detectors. The microwave detection signal may thus be representative of return loss or impedance match information. The relative position of the first and second electrodes of the electrosurgical instrument may be adjustable by the controller in the sterilization mode (i.e. when non-thermal plasma is being generated) until a set return loss threshold is reached, i.e. 8 dB, 10 dB or 12 dB.

The controller may be operable in a similar manner to select a second energy delivery profile for tissue resurfacing via thermal plasma generation. Specifically, the controller may be operable to select a second energy delivery profile for the microwave EM energy, the second energy delivery profile for the microwave EM energy being for resurfacing of tissue (via thermal plasma generation). Also, the digital microprocessor may be programmed to output a second microwave control signal for the microwave signal generator, the second microwave control signal being for setting the second energy delivery profile for the microwave EM energy.

The controller may be operable in a similar manner to select a third energy delivery profile for tissue resurfacing via non-ionising radiation (i.e. no gas). In an embodiment, the third energy delivery profile may be the same as the first energy delivery profile.

The controller may be operable in a similar manner to select an energy delivery profile for the RF EM energy. The available profiles for the RF EM energy may include a strike pulse for generating the high electric field across the flow path for striking the plasma.

The apparatus may include a movement mechanism for causing relative movement between the first electrode and second electrode, wherein the controller is arranged to communicate a control signal to the movement mechanism based on the received microwave detection signal. The movement mechanism may be mechanical, and may be manually controlled, e.g. by the operator of the instrument. The movement mechanism may comprise an actuator, e.g. lever or pull arm, located at the distal end of the instrument, e.g. a sliding or rotating mechanism that is moved by hand.

However, it is also contemplated herein to control the relative movement of the first and second electrode (i.e. setting the first and second configurations) in an automated manner, e.g. using an electromechanical mechanism. For example, in one embodiment, there may be a configuration controller arranged to automatically move the sleeve (and, if present, the jacket) and operate the gas supply.

Furthermore, the controller may be arranged to automatically operate the movement mechanism as a means for controlling the impedance match into the plasma. Reflected and forward power measurements on the microwave channel may be used to control the position of the outer catheter (or sleeve) with respect to the inner co-axial cable (or the inner electrode attached to the co-axial cable) by hand movement or by means of an electromechanical actuator (PZT actuator, a magnetostrictive actuator, stepper motor, linear motor) based on return loss measurements or impedance match.

The configuration controller may be connected to a valve to control the gas supply, e.g. to switch off the supply when the instrument moves to the second configuration and to switch it on when the instrument moves to the first configuration. The valve may be part of the instrument, e.g. integrated between the sleeve and the co-axial cable, or it may be located outside the instrument, e.g. in the gas feed.

Moreover, in combination with the microwave signal detector mentioned above, the configuration controller may be arranged to control the position of the sleeve in the first configuration when the plasma is present on the basis of the microwave detection signal to minimise the reflected microwave signal. In other words, the configuration controller comprises a feedback arrangement for fine tuning the position of the sleeve in the first configuration to facilitate efficient delivery of the plasma.

As mentioned above, the instrument is arranged to generate a non-thermal plasma for sterilisation and a thermal plasma for tissue resurfacing. With a co-axial applicator structure that has a plasma generating region with a diameter of between 3 mm and 5 mm, i.e. the inner diameter of the outer conductor within the co-axial structure has a diameter of between 3 mm and 5 mm, and a quartz tube that fits tightly inside with a wall thickness of between 0.25 mm and 1 mm, and where the outer diameter of the inner conductor is between 0.75 mm and 4 mm (allowing a space for gas to flow in the region between the inner conductor and the inner wall of the quartz tube), that a non-thermal plasma suitable for disinfection or sterilisation can be produced by operating the generator in pulsed mode with a duty cycle of less than 40%, e.g. 28%. In one embodiment, the rms power in a single microwave pulse is 50 W and the pulse ON time is 40 ms, within a total period of 140 ms, i.e. the average power delivered into the plasma is 14.28 W at 2.45 GHz. In an embodiment, this signal for generating non-thermal plasma is the aforementioned first energy delivery profile for the microwave EM energy. When an RF strike pulse is used in this configuration, the duration of the RF strike pulse is around 1 ms, and the frequency of the sinusoidal oscillations was 100 kHz. The amplitude was around 1 kV peak (707 Vrms). The RF power was less than 10% of the microwave power. The RF pulse was synchronised to the microwave burst or pulse and triggered on the rising edge of the microwave burst or pulse.

To produce thermal plasma, the duty cycle may be increased, e.g. to 50% or continuous wave (CW) and/or the rms power level may be increased, e.g. to 75 W or 100 W for this particular applicator geometry (if the geometry decreased or increased then the microwave power and the amplitude of the RF strike pulse would be adjusted accordingly). In an embodiment, this signal for generating thermal plasma is the aforementioned second energy delivery profile for the microwave EM energy. The ratio of RF to microwave power will preferably remain constant, e.g. less than 10% for non-thermal and thermal plasma.

The electrosurgical apparatus may further include a liquid feed connected to supply liquid to, or extract liquid from, the electrosurgical instrument. Also, the apparatus may be operable to supply liquid to, or extract liquid from, the area outward from the probe tip for debriding biological tissue. The liquid feed may include a container or tank for holding: (i) liquid to be injected to a treatment site, and (ii) liquid and solids (e.g. foreign objects, biological material) extracted from the treatment site. Also, the liquid feed may include a liquid supply for conveying liquid between the container and the electrosurgical instrument. Further, the liquid feed may include a control valve to controlling a direction and rate of flow of liquid to/from the electrosurgical instrument. The control valve may be part of the instrument, e.g. integrated between the sleeve and the coaxial cable (if no jacket is present), or integrated between the jacket and the sleeve (if a jacket is present), or the valve may be located outside the instrument, e.g. in the liquid supply.

Additionally, the liquid feed may further include an injection device for supplying liquid to the electrosurgical instrument via a first channel of the liquid passage, and a suction device for extracting liquid from the electrosurgical instrument via a second channel of the liquid passage. The injection device may include a compressor or a pump which forces liquid into the electrosurgical instrument at a relatively high pressure such that the liquid has enough force to dislodge foreign objects (e.g. grit, dirt, contaminants) or biological material (dead, damaged or infected tissue or cells) from a treatment site (e.g. wound, infection site, implant site). The force with which the injection device injects liquid into the electrosurgical instrument may be computer controllable, e.g. via a controller of the EM energy and gas generating equipment. The suction device may include a compressor or pump which forcibly sucks liquid (and solids) into the electrosurgical instrument's distal tip from the treatment site. For example, a first step of injecting liquid may be used to dislodge material from the treatment site (e.g. wound, infection site, implant site) and to clean the treatment site, then a second step of sucking liquid and solids may be used to suck the dirty liquid with the dislodged material out of the treatment site.

In an embodiment, the electrosurgical apparatus includes a deployment mechanism for operating a deployable debriding tool on the electrosurgical instrument. For example, the controller may be arranged to communicate a debriding signal to the deployment mechanism for moving the debriding tool between stowed and deployed positions. That is, the deployment mechanism may be computer controlled. Alternatively, however, the debridement mechanism may be manually operated, for example, via a pull wire and a handle. The deployment mechanism may be partially or entirely part of the instrument. Alternatively, the deployment mechanism may be located partly or entirely outside the instrument.

Herein, radiofrequency (RF) may mean a stable fixed frequency in the range 10 kHz to 300 MHz and microwave frequency may mean a stable fixed frequency in the range 300 MHz to 100 GHz. The RF energy should have a frequency high enough to prevent the energy from causing nerve stimulation and low enough to prevent the energy from causing tissue blanching or unnecessary thermal margin or damage to the tissue structure. Preferred spot frequencies for the RF energy include any one or more of: 100 kHz, 250 kHz, 400kHz, 500 kHz, 1 MHz, 5 MHz. Preferred spot frequencies for the microwave energy include 915 MHz, 2.45 GHz, 5.8 GHz, 14.5 GHz, 24 GHz.

BRIEF DESCRIPTION OF THE DRAWINGS

Embodiments of the invention are discussed below with reference to the accompanying drawings, in which:

Fig. 1 is a known power delivery system suitable for use with the present invention;

Fig. 2 is a schematic view of electrosurgical apparatus that is an embodiment of the invention;

Fig. 3A is a schematic cross-sectional view of an electrosurgical instrument that is an embodiment of the invention;

Fig. 3B is a schematic cross-sectional view of the electrosurgical instrument of Fig. 3A, taken along line A-A;

Fig. 4A is a schematic cross-sectional view of an electrosurgical instrument that is another embodiment of the invention in a first configuration;

Fig. 4B is a schematic cross-sectional view of the electrosurgical instrument of Fig. 4A in a second configuration; and

Fig. 5A is a schematic cross-sectional view of an electrosurgical instrument that is a further embodiment of the invention in a stowed configuration; and

Fig. 5B is a schematic cross-sectional view of the electrosurgical instrument of Fig. 5A in a deployed configuration.

DETAILED DESCRIPTION; FURTHER OPTIONS AND PREFERENCES

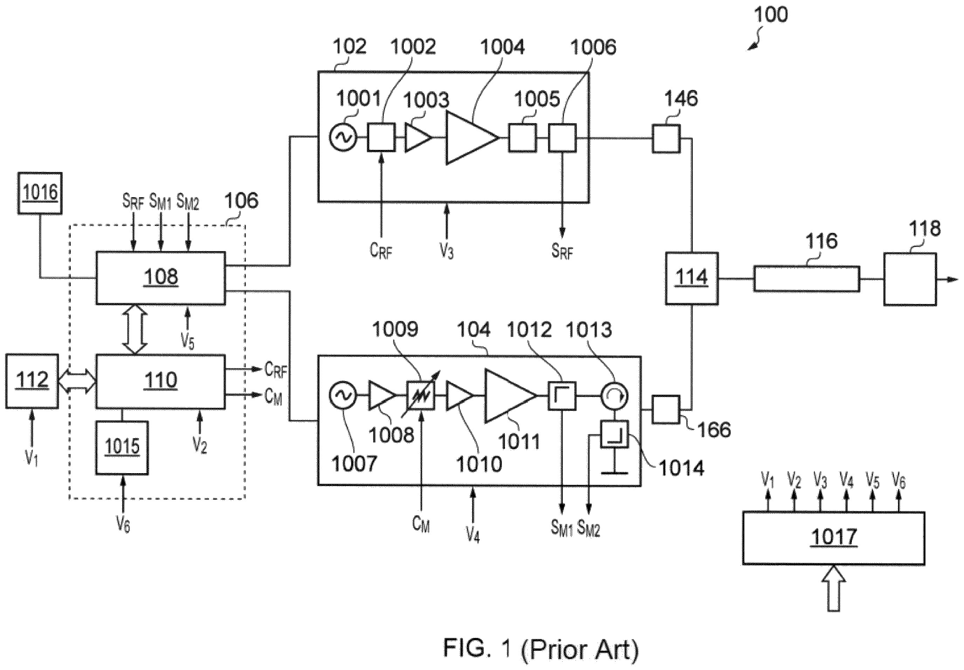

Fig. 1 shows a schematic diagram of a power delivery system 100 disclosed in WO 2012/076844, which is suitable for use in the present invention.

The system 100 comprises an RF line-up 102 and a microwave line-up 104, which form parts of a RF channel and a microwave channel respectively.

The RF line-up 102 contains components for generating and controlling an RF frequency electromagnetic signal at a power level suitable for striking a plasma, as described below. In this embodiment, it includes an RF oscillator 1001, a power controller 1002, an amplifier unit (here comprising a driver amplifier 1003 and a power amplifier 1004), a transformer 1005 and an RF signal detector 1006.

The microwave line-up 104 contains components for generating and controlling a microwave frequency electromagnetic signal at a power level suitable for treating biological tissue (e.g. sterilizing and resurfacing). In this embodiment it includes a phase locked oscillator 1007, a signal amplifier 1008, an adjustable signal attenuator (e.g. an analogue or digital PIN diode based attenuator attenuator) 1009, an amplifier unit (here a driver amplifier 1010 and a power amplifier 1011), a forward power coupler 1012, a circulator 1013 and a reflected power coupler 1014. The circulator 1013 isolates the forward signal from the reflected signal to reduce the unwanted signal components present at the couplers 1012, 1014, i.e. it increases the directivity of the couplers. The circulator also protects the transistors within the high power output stage, e.g. the power GaN or GaAs transistors. It is preferable for the isolation between ports 1 to 3, 2 to 1 and 3 to 2 to be as high as possible, i.e. greater than 15 dB, or more preferably more than 20 dB.

The RF line-up 102 and microwave line-up 104 are in communication with a controller 106, which may comprise signal conditioning and general interface circuits 108, a microcontroller 110, and watchdog 1015. The watchdog 1015 may monitor a range of potential error conditions, which could result in the system not performing to its intended specification, i.e. the system delivers the wrong dosage of energy into patient tissue due to the output or the treatment time being greater than that demanded by the user. The watchdog 1015 comprises a microprocessor that is independent of the microcontroller 110 to ensure that microcontroller is functioning correctly. The watchdog 1015 may, for example, monitor the voltage levels from DC power supplies or the timing of pulses determined by the microcontroller 110. The controller 106 is arranged to communicate control signals to the components in the RF line-up 102 and microwave line-up 104. In this embodiment, the microprocessor 110 is programmed to output an RF control signal $C_{RF}$ and a microwave control signal $C_M$ for the power controller 1002 and the adjustable signal attenuator 1009 respectively. These control signals are used to set the energy delivery profile of the RF EM radiation and the microwave EM radiation output from the RF line-up 102 and microwave line-up 104 respectively. In particular, the power controller 1002 and the adjustable signal attenuator 1009 are capable of controlling the power level of the output radiation. Moreover, the power controller 1002 and the adjustable signal attenuator 1009 may include switching circuitry capable of setting the waveform (e.g. pulse width, duty cycle, and amplitude, etc.) of the output radiation.

The microprocessor 110 is programmed to output the RF control signal $C_{RF}$ and the microwave control signal $C_M$ based on signal information from the RF signal detector 1006 and forward and reflected power couplers 1012, 1014. The RF signal detector 1006 outputs a signal or signals $S_{RF}$ which are indicative of the voltage and current (and optionally the phase between the voltage and current) of the RF EM radiation on the RF channel. In this embodiment, the RF and microwave generator may be controlled by measurement of phase information only, which can be obtained from either the RF channel (from sampled current and voltage information) or the microwave channel (from sampled forward and reflected power information). The forward power coupler 1012 outputs a signal $S_{M1}$ indicative of the forward power level and the reflected power coupler 1014 outputs a signal $S_{M2}$ indicative of the reflected power level. The signals $S_{RF}$, $S_{M1}$, $S_{M2}$ from the RF signal detector 1006 and the forward and reflected power couplers 1012, 1014 are communicated to the signal conditioning and general interface circuits 108, where they are adapted to a form suitable for passing to the microprocessor 110.

A user interface 112, e.g. touch screen panel, keyboard, LED/LCD display, membrane keypad, footswitch or the like, communicates with the controller 106 to provide information about treatment to the user (e.g. surgeon) and permit various aspects of treatment (e.g. the amount of energy delivered to the patient, or the profile of energy delivery) to be manually selected or controlled, e.g. via suitable user commands. The apparatus may be operated using a conventional footswitch 1016, which is also connected to the controller 106.

The RF and microwave signals produced by the RF line-up 102 and microwave line-up 104 respectively are input to a signal combiner 114, which conveys the RF and microwave EM radiation separately or simultaneously along a cable assembly 116 to the probe 118. In this embodiment, the signal combiner 114 comprises a duplexer-diplexer unit that allows energy at microwave and RF frequencies to be transmitted along cable assembly 116 (e.g. a coaxial cable) to a probe (or applicator) 118, from which it is delivered (e.g. radiated) into the biological tissue of a patient into the instrument channel of a scope, e.g. an endoscope or another surface.

The signal combiner 114 also permits reflected energy, which returns from the probe 118 along cable assembly 116, to pass into the microwave and RF line-ups 102, 104, e.g. to be detected by the detectors contained therein. As explained below, the apparatus may include a low pass filter 146 on the RF channel and a high pass filter 166 on the microwave channel, so that only a reflected RF signal enters the RF line-up 102 and only a reflected microwave signal enters the microwave line-up 104.

Finally, the apparatus includes a power supply unit 1017 which receives power from an external source 1018 (e.g. mains power) and transforms it into DC power supply signals $V_1$-$V_6$ for the components in the apparatus. Thus, the user interface receives a power signal $V_1$, the microprocessor 110 receives a power signal $V_3$, the RF line-up 102 receives a power signal $V_3$, the microwave line-up receives a power signal $V_4$, the signal conditioning and general interface circuits 108 receives a power signal $V_5$, and the watchdog 1015 receives a power signal $V_6$.

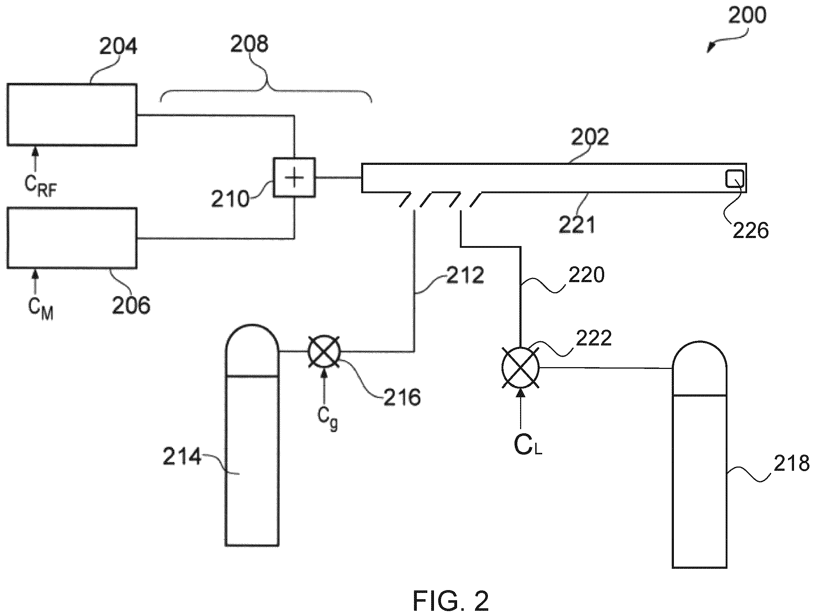

Fig. 2 shows a schematic diagram of electrosurgical apparatus 200 that is an embodiment of the invention. The apparatus 200 comprises an electrosurgical instrument 202 capable of delivering plasma or non-ionising electromagnetic (EM) radiation from its distal end. Examples of the structure of the instrument 202 are described below.

The instrument 202 is connected to a power delivery system, which may be as described with reference to Fig. 1. However, in the embodiment of Fig. 2, the power delivery system comprises a radiofrequency (RF) radiation source 204 and a microwave radiation source 206 which are connected to deliver power to the proximal end of the instrument 202 via a feed structure 208. The feed structure 208 may include a signal combiner unit 210 as discussed above. The RF source 204 and the microwave source 206 may be arranged to output an RF signal and a microwave signal respectively based on control signals $C_{RF}$ and $C_M$ from a controller (not shown).

The instrument 202 is also connected to receive a gas, e.g. from a pressurised gas source 214 via supply line 212. A control valve 216 on the supply line 212 may be arranged to control the flow of gas received by the instrument 202, e.g. based on a control signal $C_g$ from the controller. It may be desirable to activate the gas control valve and/or flow controller prior to activating the RF and/or microwave energy sources in order to ensure that gas is present when said energy sources are activated as it is necessary for gas to be present in the plasma forming region before plasma can be generated. It may be preferable to include a gas sensor in the plasma forming region and the signals from this sensor used to control the gas flow valves. This system also helps control gas utilisation and prevents the patient from filling up with argon (or other) gas.

The RF and microwave measurement information may also be used to control the gas controller, i.e. the gas control valve may be closed when RF and/or microwave power cannot be detected using voltage/current and/or forward/reflected power monitoring circuits within the generator. It may be preferable to wait for a set period of time, i.e. 20 ms or 200 ms before shutting off the gas supply. This arrangement acts as a safety feature and as a means of controlling gas usage.

The instrument 202 is also connected to receive a liquid (e.g. water, saline), e.g. from a tank or container 218 via supply line 220. The instrument 202 may also be configured to provide liquid and solids (e.g. biological material and foreign objects dislodged from a treatment site) to the container 218 via supply line 220. A control valve 222 on the supply line 220 may be arranged to control the flow of liquid received by the instrument 202 from the container 218, and/or the flow of liquid (and solids) received by the container 218 from the instrument 202. The control valve 222 may be operable to control the flow direction and/or flow rate. For example, the direction and magnitude of flow may be based on a control signal $C_L$ from the controller. In this way, the instrument 202 may inject liquid (e.g. water, saline) from the container 218 into a treatment site at a distal end of the instrument 202. This liquid may be used to debride biological tissue at the treatment site (e.g. a wound, infection site, implant site). Additionally or alternatively, debridement may involve extracting liquid, foreign objects and/or biological material (e.g. tissue, cells) from the treatment site and into the container 218. The container 218 and supply line 220 may be partitioned into two or more zones, wherein a first zone is for providing liquid from the liquid container 218 to the instrument 202, and a second zone is for providing liquid and solids to the container 218 from the instrument 202. The first zone may be separated from the second zone such that solids and/or liquids in one zone are prevented from entering the other zone.

The instrument 202 may comprise an outer sleeve or jacket 221 which carries a coaxial cable and gas from its proximal end to the distal end for delivering plasma (e.g. non-thermal and thermal) or non-ionising radiation into biological tissue at a treatment site at or just beyond a distal end of the instrument 202. Also, the sleeve or jacket 221 may transport liquid (including solids) between its proximal end and distal end for the purposes of debriding. Furthermore, the instrument 202 may include an abrasive region 226 on a distal outer surface for debriding biological tissue in the treatment site. That is, the abrasive region 226 may be used to scrape away dead, damaged of infected tissue, and/or foreign objects (e.g. grit, dirt, contaminants) from a treatment site (e.g. a wound, infection site, implant site). The abrasive region 226 may cover the whole circumference of the instrument distal tip (e.g. it may be roughly annular or ring shaped) or cover only a portion of it (e.g. as shown in Fig. 2). The abrasive region 226 is shown as being substantially rectangular but, in some other embodiments, it may be a different shape, e.g. circular, oval, triangular, regular or irregular. The abrasive region 226 may be positioned on a side surface or end face of the probe tip.

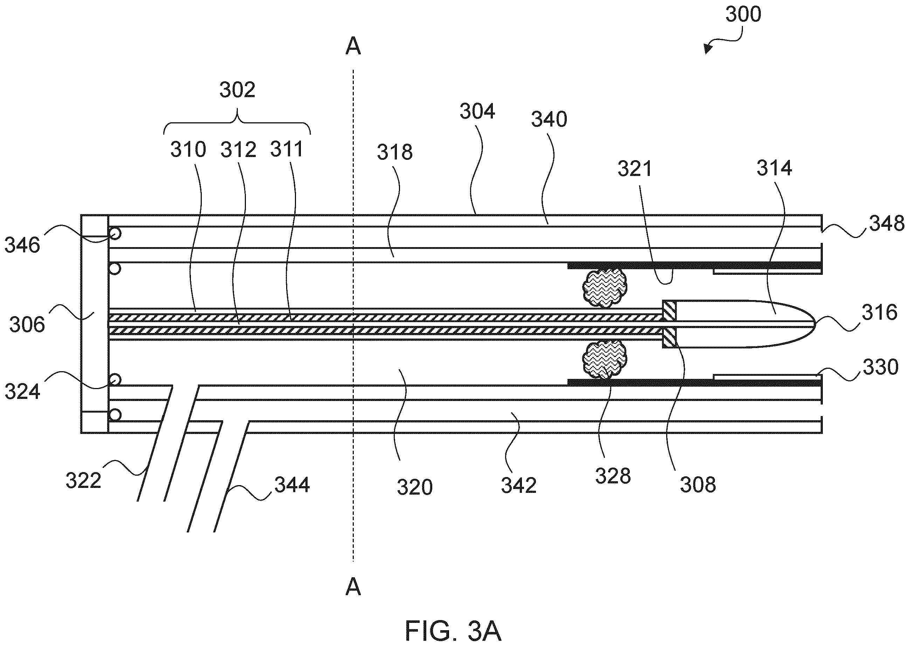

Fig. 3A shows a first embodiment of an electrosurgical instrument 300 according to the invention. The instrument 300 comprises an elongate probe made up of a central coaxial cable 302 surrounded by a tubular sleeve 318. The proximal end of the coaxial cable 302 (shown on the left in Fig. 3A) terminates at a suitable connector 306 that is adapted to connect to the feed structure that supplied the RF and microwave signals. The coaxial cable 302 conveys the RF and microwave signals to the distal end of the instrument (on the right in Fig. 3A).

The distal end of the coaxial cable 302 terminates at an insulating element 308 such as a glass bead or ceramic disc positioned between the body of the coaxial cable and the cylindrical cap to prevent shorting or breakdown from occurring. Alternatively, the dielectric 311 of the cable 302 may extended by e.g. 0.1mm to 0.2 mm past the outer conductor 310 of the cable 302. The outer conductor 310 of the coaxial cable stops at the insulating element 308, but the inner conductor 312 of the cable 302 continues through the insulating element 308 and protrudes beyond the insulating element 308 for a length selected (using simulations) to give best impedance match for tissue resurfacing (aka re-epithelialisation). The protruding length is surrounded by a cylindrical ceramic (or other suitable dielectric or magnetic material) cap 314, which terminates at its distal end in a dome 316, e.g. a hemisphere. The inner conductor 312 protrudes slightly from the dome 316. The inner conductor 312 and cylindrical cap function as a first electrode of the instrument.

The sleeve 318 surrounds the coaxial cable 302 to define an annular space 320 between the outer surface of the coaxial cable 302 and the inner surface of the sleeve 318. Radial support elements or spacers (not shown) may be used to locate the coaxial cable 302 within the sleeve. The annular space 320 may be used to transport gas to the distal end of the instrument. The base piece 318 has a port 322 in a side surface thereof that is connected to the gas supply line. A gas tight seal 324, which may be an O-ring or the like, is provided at the join between the sleeve 318 and the connector 306 in order to minimise the escape of gas. Gas introduced into the port 322 therefore flows along the annular space 320 to exit the instrument at its distal end.

The sleeve 318 has an electrically conductive inner surface 321 along a length thereof leading up to its distal end. For example, the sleeve may comprise a stainless steel shaft with a polyimide liner on its outer surface. Its electrically conductive inner surface 321 is electrically connected to the outer conductor 310 of the coaxial cable 302. In this embodiment, this is done by means of an electrically conductive mesh 328 mounted within the annular space 320. The mesh is porous, and therefore permits the gas to flow through it whilst also providing an electrical connection. This could also be achieved using a spring or a plurality of small wires electrically connected, i.e. soldered or crimped or trapped, to one or both surfaces of conductors or electrodes 310 and 321. Providing at least two, ideally at least four, circumferential contact points around the circumference of the conductor(s) can ensure a good enough electrical contact for the microwave energy to propagate unimpaired. It may also be possible and preferable to put a plurality of dents or a partial crimp (e.g. around one half) in/on one of the conductors in order to make the necessary electrical contact needed whilst also enabling the gas to flow onto the plasma generating region or the distal end of the device where plasma is formed.

The electrically conductive inner surface 321 of the sleeve is further covered by an insulating tube 330 (e.g. made of quartz, ceramic or the like) along a distal length thereof that can overlap longitudinally with the cylindrical cap 314. The electrically conductive inner surface 321 and insulating tube 330 function as a second electrode of the instrument.

The tubular sleeve 318 is surrounded by an outer jacket 340 which surrounds the sleeve 318 to define an annular space 342 between the outer surface of the sleeve 318 and the inner surface of the jacket 340. As before, radial support elements or spacers (not shown) may be used to locate the sleeve 318 within the jacket 340. The annular space 342 may be used to transport liquid to the distal end of the instrument (e.g. to a treatment site at the distal end). Additionally, the annular space 342 may be used to transport liquid (e.g. water, saline) and/or solids (e.g. foreign objects (e.g. grit), biological material (e.g. cells)) from the distal end (e.g. from a treatment site at the distal end) to a proximal end of the instrument. The jacket 340 has a port 344 in a side surface thereof that is connected to the liquid supply line.

A liquid tight seal 346, which may be an O-ring or the like, is provided at the join between the jacket 340 and the connector 306 in order to minimise the escape of liquid. Liquid introduced into the port 344 therefore flows along the annular space 342 to exit the instrument at its distal end. Specifically, one or more outlets 348 are provided in a distal end of the annular space 342 such that liquid may flow out of the instrument 300 and into a treatment site at a distal end of the instrument. Additionally, liquid and solids may be sucked into the annular space 342 via the outlets 348. The outlets 348 may be generally circular, and may be substantially uniformly circumferentially spaced. However, it is to be understood that any shape, number or distribution of outlets 348 may be possible provided that they permit liquid to flow out of the instrument's distal end, and permit liquid and solids (e.g. foreign objects (e.g. grit, dirt, contaminants) and biological material (e.g. tissue, cells)) to flow into the distal end.

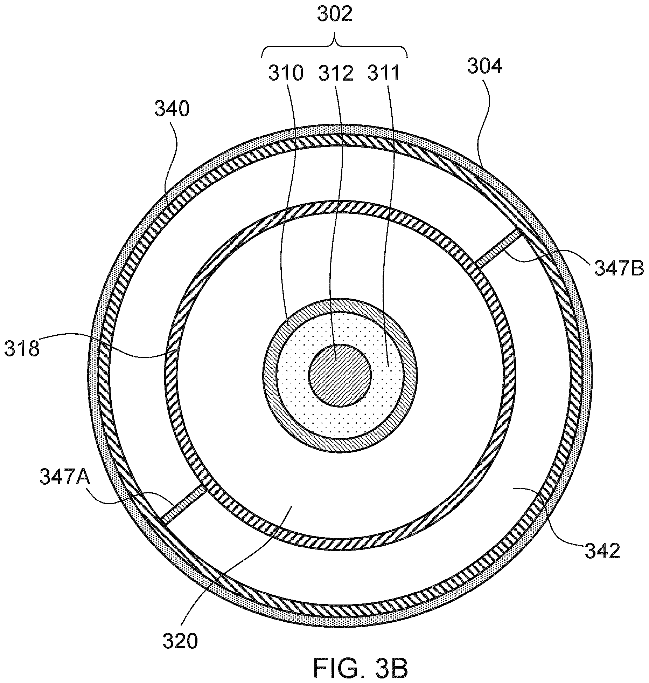

Fig. 3B is a cross section view taken along the line A-A in Fig. 3A. As seen from Fig. 3B, the annular space 342 is divided or partitioned into multiple channels. In the embodiment shown, dividing elements or structures or dividers 347A and 347B divide the annular space 342 into two channels. In this way, one of the channels can be used to transport liquid (and solids) from the treatment site, into the distal end of the instrument, and back to a container or tank (e.g. container 218) for storage and/or disposal. Also, the other one of the channels can be used to transport liquid from the container or tank through the instrument, out of the distal end, and into the treatment site. As such, liquid can be injected to the treatment site, for example, to debride a wound or infection/implant site in the treatment site, then dirty liquid containing solids (e.g. foreign objects (e.g. grit or dirt) and biological matter (e.g. cells or tissue)) can be sucked out of the treatment site to clean the wound or infection/implant site. It is to be understood that in some other embodiments, the annular space 342 may be divided into more than two channels, e.g. 4, 6, 8 or 10 channels. In this case, one or more channels may introduce liquid to a treatment site, and/or one or more channels may extract liquid (and solids) from the treatment site. Additionally, in some embodiments, the annular space 342 is not divided and, instead, provides a single channel which is used both for introducing liquid into the treatment site and for extracting liquid or solids from the treatment site.

The jacket 340 is provided with an outer protective sheath 304, e.g. formed of polyimide or the like. The protective sheath 304 terminates at its distal end. In an embodiment, the termination may include an annular structure made from a suitable insulator, e.g. a low loss microwave ceramic, PTFE, PEEK, Nylon or the like.

The instrument is arranged to generate a plasma (e.g. non-thermal or thermal plasma) in an area outward from the probe tip (e.g. a treatment site at or just beyond the probe tip) by taking the following steps:

- supply gas to the distal region of the instrument (i.e.

to the region between the quartz tube 330 and cylindrical cap 314),
- sending a pulse of RF energy through the coaxial cable to strike a plasma in the gas at the distal region by generating a high electric field in the region, and
- sending a pulse of microwave energy through the coaxial cable to sustain or maintain the plasma to ensure that appropriate treatment takes place.

The RF pulse may be automatically triggered by a characteristic (e.g. the rising edge) of the microwave pulse, so that the strike and sustain pulses are always synchronised. The RF pulse is arranged to have a voltage suitable for setting up an electric field for striking the plasma. The voltage may be between 150 V and 1500 V peak, more preferably between 250 V and 750 V peak. The frequency of the RF pulses may be between 100 kHz and 1 MHz, and may comprise a window or burst of sinusoidal waveform or signals that is time-gated (e.g. based on the detected microwave pulse), e.g. to have a duration of between 0.5 $\mu$s and 10 ms.

The delivered microwave power may be monitored (e.g. by measuring forward and reflected microwave signals) in order to check the status of the plasma.

In the embodiment above, the plasma is struck by the RF signal. In other embodiments, the plasma may be struck by the microwave signal only, because the close proximity between the inner and outer conductors enables a high electric field to be generated from the microwave signal. For example, if it is possible to deliver 25 W of CW microwave power to the distal end of the instrument then this may create a high enough electric field. One possible means of striking plasma using the microwave field is to decrease the distance between the two conductors within the plasma generating region at the time plasma is struck and then increase the distance again once it has been struck in order to create the optimal environment (impedance) for plasma to be sustained.

The electrosurgical instrument 300 may provide a wound treatment apparatus. Initially, the instrument 300 may be used to perform debriding of the wound. For instance, an operator may instruct a controller (e.g. controller 106, via user interface 112) to activate a liquid control valve (e.g. valve 222) in order to inject liquid (e.g. water, saline) from a container (e.g. 218), via a supply line (e.g. 220) into a distal end of the instrument 300 at port 344. The liquid is then transported in annular space 342 and exits the instrument at the distal tip via outlets 348. Thereby output liquid is injected into a treatment site located outward from the distal end, e.g. at or just beyond the distal end. The injected liquid can be used to debride a wound in the treatment site. For example, the injected liquid may dislodge dead, damaged, or infected tissue from the wound. Also, the injected liquid may dislodge foreign objects (e.g. dirt, grit, contaminants) from the wound. This operation may be sufficient to complete the debriding process. However, in some circumstances, further operations may be preferably or necessary. For example, sequentially or simultaneously, the operator may cause the apparatus to further control the liquid control valve such that liquid (and solids) are sucked from the wound and into the distal end of the instrument 300 via the outlets 348. This mix of liquids and solids may then be transported through the instrument in annular space 343, and back to the container (e.g. container 218). In an embodiment, the same fluid passages are used, first, to inject liquid into the wound and, second, to extract liquid and solids from the wound. However, as explained above with reference to Fig. 3B, the annular space 342 may be partitioned into multiple channels such that different channels are used for (sequentially or simultaneously) injecting liquid and extracting liquid (with solids). Also, the supply line, liquid control valve and container may have separate spaces or zones for injecting liquid and extracting liquid (with solids).

After debriding, the instrument 300 may then be used to sterilize biological tissue, for example, to sterilize the wound in the treatment site. Specifically, the operator (e.g. via controller 106 and user interface 112) may control an RF line up (e.g. line-up 102) and a gas feed (e.g. gas container 214, valve 216 and gas supply 212) to combine gas with a strike signal at the distal end of the instrument (e.g. between the first and second electrodes) in order to strike a plasma. Then, a microwave line-up (e.g. microwave line-up 104) may be used to sustain a non-thermal plasma.

For example, a non-thermal plasma suitable for disinfection or sterilisation can be produced by operating the MW generator in pulsed mode with a duty cycle of less than 40%, e.g. 28%. In one embodiment, the rms power in a single microwave pulse is 50 W and the pulse ON time is 40 ms, within a total period of 140 ms, i.e. the average power delivered into the plasma is 14.28 W at 2.45 GHz. When an RF strike pulse is used in this configuration, the duration of the RF strike pulse may be around 1 ms, and the frequency of the sinusoidal oscillations may be 100 kHz. The amplitude may be around 1 kV peak (707 Vrms). The RF power can be less than 10% of the microwave power. The RF pulse can be synchronised to the microwave burst or pulse and triggered on the rising edge of the microwave burst or pulse.

In this way, non-thermal plasma can be generated at the distal end of the instrument 300, and this non-thermal plasma can be delivered to a treatment site at or just beyond the distal end. According, the non-thermal plasma can be directed to the newly debrided wound in order to sterilise or clean the wound, e.g. by killing bacteria in the wound. This process of wound sterilisation is useful in reducing the chances that the wound will become infected which, in turn, can improve the chances that the wound will heal.

After debriding and sterilisation, the instrument 300 may then be used to resurface biological tissue, for example, to resurface the wound in the treatment site. Specifically, the operator (e.g. via controller 106 and user interface 112) may control the microwave line-up (e.g. microwave line-up 104) and gas feed (e.g. gas container 214, valve 216 and gas supply 212) to form a thermal plasma. As before, this step may follow a step striking the plasma. To produce thermal plasma, the duty cycle may be increased, e.g. to 50% or continuous wave (CW) and/or the rms power level may be increased, e.g. to 75 W or 100 W (the microwave power and the amplitude of the RF strike pulse would need to be adjusted based on the precise geometry/dimensions of the instrument 300). The ratio of RF to microwave power will preferably remain constant, e.g. less than 10% for non-thermal and thermal plasma.

In this way, thermal plasma can be generated at the distal end of the instrument 300, and this thermal plasma can be delivered to a treatment site at or just beyond the distal end. According, the thermal plasma can be directed to the newly sterilised wound in order to resurface the wound. This process of wound resurfacing is useful in accelerating wound healing compared to simply waiting for natural wound healing to occur.

In view of the above, the electrosurgical instrument 300 provides a wound treatment apparatus which can perform debriding, sterilising and resurfacing of a wound. It is to be understood that transitioning from sterilizing to resurfacing involves transitioning from producing/applying non-thermal plasma to producing/applying thermal plasma. It is further to be understood that this transition from non-thermal to thermal plasma may be performed by increasing the duty cycle and/or increasing the power of the pulsed microwave energy signal used to form the plasma. For example, a duty cycle below 40% may be used to produce non-thermal plasma, whereas a duty cycle above 50% may be used to produce thermal plasma. Additionally or alternatively, a pulse power of below 50W (or an average power of below 15W) may be used to produce non-thermal plasma, whereas a pulse power of above 75W (or an average power of above 30W) may be used to produce thermal plasma.

It is to be understood that whilst the electrosurgical instrument of Figs. 3A and 3B can be used in wound treatment, the instrument can also find utility in other applications, such as, in treating infection sites, for example, in the ear and urinary tract. For instance, the liquid passage 342 may be used to extract infected tissue and/or mucus from the infection site. Further, the liquid passage 342 may be used to extract infected tissue from a medical implant (e.g. metal implant) and a corresponding bodily implant site. Subsequently, the infection site, the implant site and/or the implant can be sterilized using non-thermal plasma.

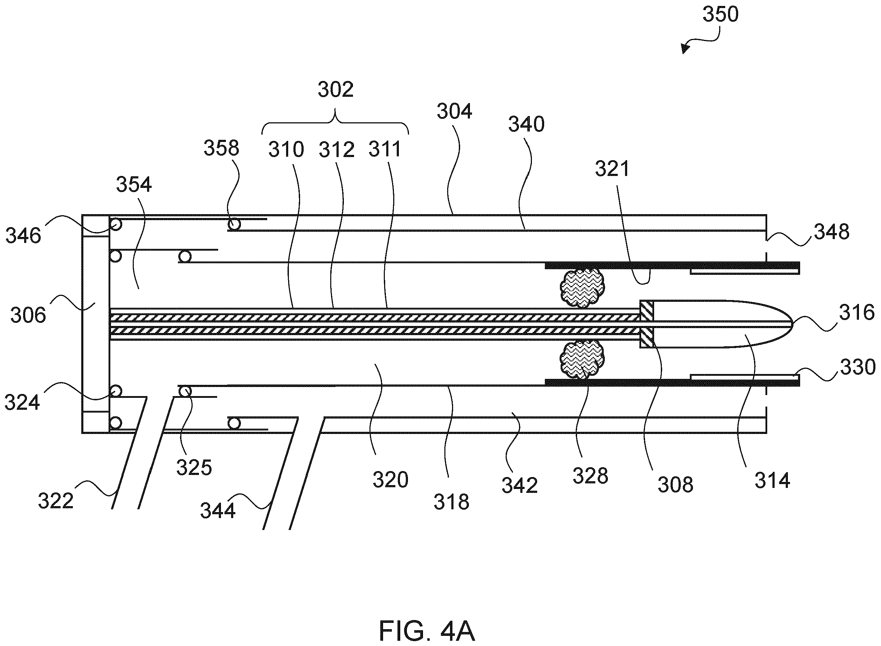

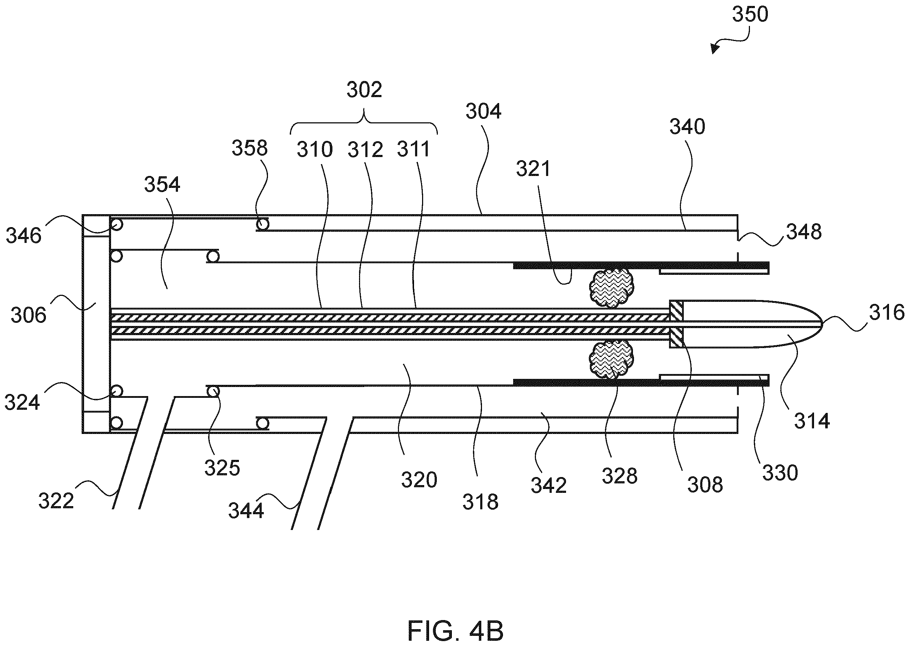

Figs. 4A and 4B are schematic cross-section views of an electrosurgical instrument 350 that is another embodiment of the invention. Components in common with Fig. 3A are given the same reference numbers and are not described again.

The embodiment in Figs. 4A and 4B differs from the embodiment of Figs. 3A and 3B in that the first electrode and the second electrode are movable relative to each other.

In this embodiment, the sleeve 318 and jacket 340 are arranged to slide in a longitudinal direction relative to the coaxial cable 302. To achieve this, the sleeve 318 is slidably mounted in a telescopic manner within a proximal base piece 354. An O-ring 325 may be fitted at the sliding interface to maintain a fluid (e.g. gas) tight seal. Also, the jacket 340 is slidably mounted in a telescopic manner within the proximal base piece 354. An O-ring 358 may be fitted at the sliding interface to maintain a fluid (e.g. liquid) tight seal. A pull wire (not shown) may extend through the connector 306 to assist positioning of the sleeve 318 and the jacket 340 relative to the coaxial cable. The pull wire may be manually operated, or may be connected to an automated control mechanism, e.g. a stepper motor or linear motor, which can automatically control the position of the sleeve 318 and the jacket 340, e.g. on the basis of a control signal from the controller. The sleeve 318 and the jacket 340 may move together as one single unit.

The slidable sleeve and jacket permits the instrument to adopt two configurations. In a first configuration, as shown in Fig. 4A, the electrically conductive inner surface 321 of the sleeve 318 is longitudinally in line with the cylindrical cap 314. This configuration sets up a region of high impedance which exhibits a high electric field when the RF or microwave signal is supplied to the instrument. In this configuration, the instrument may be adapted to deliver plasma, e.g. thermal plasma for resurfacing or non-thermal plasma for sterilisation, from the distal end of the probe. This mode of operation corresponds to the device shown in Fig. 3A.

The microprocessor (e.g. microprocessor 110) may be arranged to output a control signal to adjust the position of the sliding sleeve and jacket relative to the coaxial cable based on the detected return loss or impedance mismatch that is determined in the controller from the microwave detection signal. This control may be done when plasma is being generated e.g. to maintain a pre-set required match or return loss, e.g. 10 dB (90% of the microwave energy is delivered into the plasma).

In a second configuration, as shown in Fig. 4B, the sleeve 318 and the jacket 340 are slid back relative to the coaxial cable 302 to expose a length of the cylindrical cap 314 at the distal end of the device. The exposed end functions as a radiating monopole microwave antenna. In this configuration, a microwave signal is supplied to the coaxial cable in the absence of gas. The microwave signal is emitted at a non-ionising radiation field. The levels of non-ionising microwave power delivered at the distal radiating monopole may be between 2.5 W and 50 W continuous wave power; the level can be dependent on the characteristics of the tissue being resurfaced, for example, the depth of the treatment site (e.g. wound or infection/implant site). The power level also depends on the properties of the microwave transmission cable used to deliver the microwave energy from the generator to the applicator or antenna.

The microwave energy may be delivered as a sequence of pulses or a burst of microwave energy, whereby the mechanical force follows or is embedded within the burst of microwave coagulation energy. For example, one activation profile may comprise applying 10 W of microwave power for 10 seconds.

The distal end of the cylindrical cap 314 may terminate in an electrically conductive dome, which helps to ensure that the power density in the area outward of the probe tip is not too highly concentrated at the distal end of the cylindrical cap 314.

In view of the above, an addition to, or instead of, resurfacing via thermal plasma (i.e. an ionised gas), the instrument 300 may be used to produce non-ionised microwave energy or radiation for tissue resurfacing. In this case, the apparatus can be controlled (e.g. via controller 106 and user interface 112) to shut off the gas feed (e.g. gas container 214, valve 216 and gas supply 212) such that gas is not provided to the distal end of the instrument 300. Additionally, the microwave line-up (e.g. microwave line-up 104) can be controlled (e.g. via controller 106 and user interface 112) to provide the non-ionising radiation field at the distal tip. In an embodiment, the same microwave signal used for producing non-thermal plasma (with gas) may be used to generate the non-ionising radiation field (without gas). This type of microwave signal or microwave energy profile may be desirable since it is weaker compared to, for example, the signal used to generate thermal plasma. A weaker signal can be beneficial for resurfacing since a stronger signal may result in a level of coagulation (e.g. shallow or deep) that may hinder healing. That said, as discussed above, it is to be understood that in some other embodiments a different form of microwave energy profile may be used, e.g. a pulsed signal with a duty cycle above 40% and/or a pulse power of above 50W (or an average power of above 15W).

In view of the above, the electrosurgical instrument 350 provides a wound treatment apparatus or device which can perform wound debriding and sterilising and, also, resurfacing via two separate and distinct mechanisms: thermal plasma and non-ionising microwave energy.

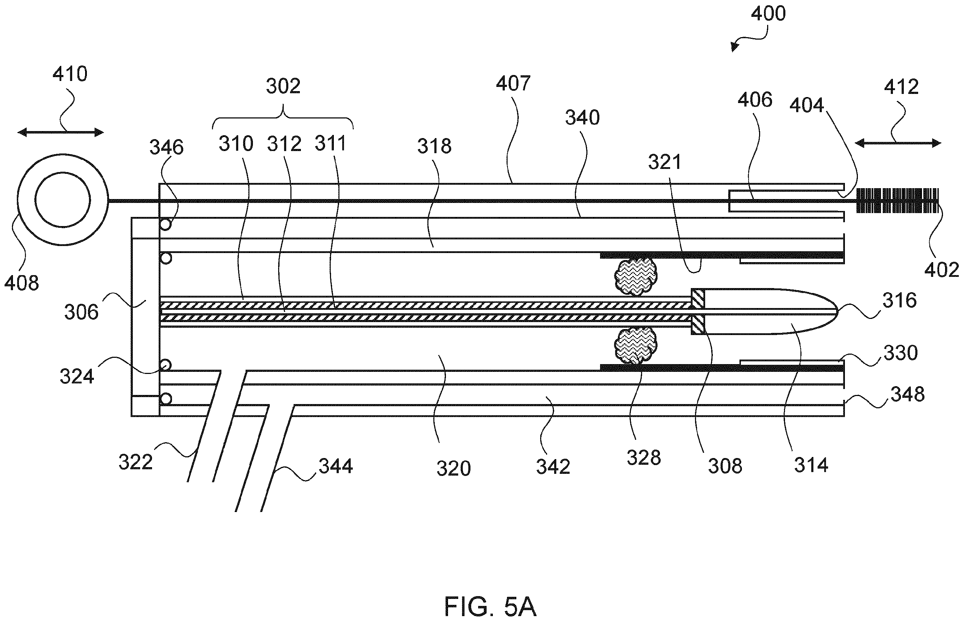

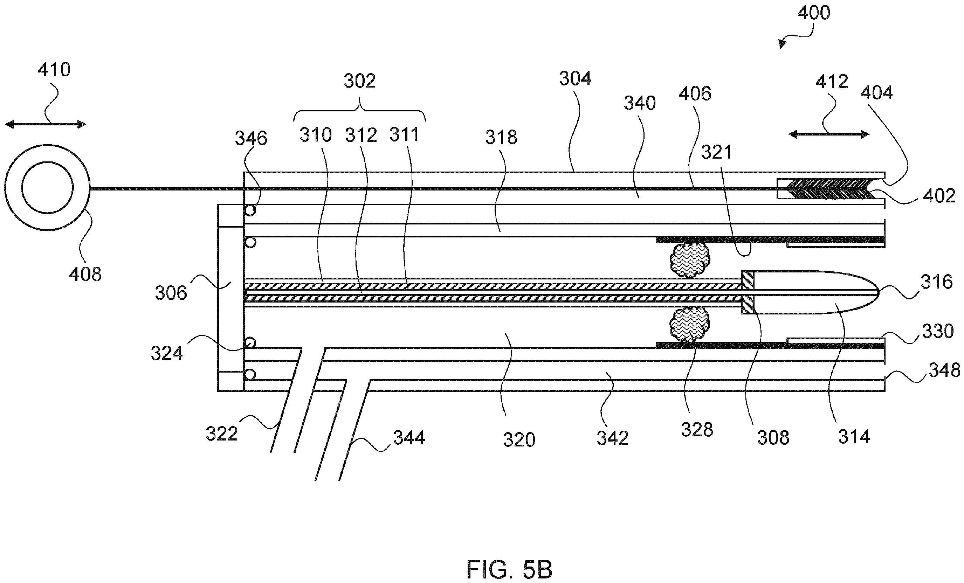

Figs. 5A and 5B are schematic cross-section views of an electrosurgical instrument 400 that is a further embodiment of the invention. Components in common with Fig. 3A are given the same reference numbers and are not described again.

The embodiment in Figs. 5A and 5B differs from the embodiment of Fig. 3A in that the instrument includes a deployable debriding tool 402. Specifically, the instrument 400 comprises a cavity, recess or holder 404 for receiving the debriding tool 402. In the embodiment of Figs. 5A and 5B, the holder 404 is in a distal end of the instrument 400, and has an opening on a distal end face which faces in substantially the same direction that plasma and non-ionising radiation is emitted. However, in some other embodiments, it is to be understood that the holder 404 could be positioned elsewhere on the instrument 400. The instrument 400 further includes a pull wire 406 which extends from the debriding tool 402 to a handle 408 at the proximal end of the probe. As shown in Figs. 5A and 5B, the pull wire 406 may be housed within a guide structure 407 which is attached to an outer surface of the instrument 400 and protects the wire from becoming snagged on objects during use. However, in another embodiment, the guide structure 407 may be integrated with the jacket 340 or sleeve 318 such that the outer profile of the instrument 400 is unchanged compared to the embodiments of Figs 3A-4B. In any case, the handle 408 may be operated by a user to move the debriding tool 402 relative to the holder 404. That is, the user can move the handle 408 longitudinally (as indicated by the arrow 410) relative to the instrument 400 in order to cause a corresponding longitudinal movement (as indicated by the arrow 412) of the debriding tool 402 relative to the holder 404. In this way, the handle 408 can be used to move the debriding tool 402 between a stowed position (shown in Fig. 5B), in which the debriding tool 402 is enclosed within the holder 406, and a deployed position (shown in Fig. 5A), in which the debriding tool 402 protrudes into the area outward from the probe tip for debriding biological tissue. In an embodiment, the holder 404 is dimensioned such that, in the stowed position, the debriding tool 402 is completely enclosed within the holder 402, i.e. the tool does not protrude at all from the holder. Also, in an embodiment, the pull wire 406 is dimensioned such that the debriding tool 402 is completely spaced from the holder 402, i.e. the whole tool protrudes from the holder. However, in some other embodiment, the various components may be dimensioned such that the tool only partially protrudes from the holder in the deployed position.

In Figs. 5A and 5B the debriding tool comprises a brush having a plurality of resiliently deformable bristles. When in the deployed position, the bristles may be used to remove dead, damaged or infected tissue from a treatment site (e.g. wound, infection/implant site, implant), e.g. via a brushing or scrapping action. Also, the bristles may be used to remove foreign objects (e.g. grid, dirt, contaminants) from the treatment site, e.g. via a brushing or scrapping action. However, in some other embodiments, the debridement tool may be something other than a brush, such as, for example, a deployable member (e.g. a pad) with an abrasive coating or surface, or a deployable member coated with a gauze type material. In such cases, the method of debriding using the debriding tool is the same as for the brush.

In the embodiment of Fig. 5A and 5B, the deployment mechanism for the deployable tool 402 includes the pull wire 406 and handle 408. As such, the deployment mechanism of Fig. 5A and 5B is a manual deployment mechanism. However, it is to be understood that in some other embodiments, an automatic or computer controlled deployment mechanism may be provided. For instance, such an automatic deployment mechanism may include an electromechanical actuator (PZT actuator, a magnetostrictive actuator, stepper motor, linear motor) operatively coupled to a controller (e.g. controller 106), and the actuator may be configured in use to move the debriding tool 402 between the stowed and deployed positioned based on control signals from the controller. The actuator may mechanically move the tool using a pull wire similar to the pull wire 406.

The embodiment of Figs. 5A and 5B has both the deployable debriding tool 402 and the liquid passage 342 for injecting/extracting debriding liquid. As such, the embodiment of Fig. 5A and 5B has two separate and distinct mechanisms for debriding a treatment site (e.g. a wound, infection/implant site, implant). However, it is to be understood that where the deployable tool 402 is provided, the liquid passage 342 and all associated mechanisms for injecting and extracting liquid are optional, i.e. they may not be present.

It is to be understood that one or more features from one of the above-described embodiments may be combined with one or more features from one or more others of the above-described embodiments to form a new embodiment falling within the scope of the appended claims. For example, the embodiment of Fig. 2 includes an abrasive region 226 at a distal end of the instrument for debriding a treatment site (e.g. wound, infection/implant site). It is to be understood that any one or more of the embodiments of Figs. 3A, 3B, 4A, 4B, 5A and 5B may also include an abrasive region 226. Additionally, the above-described embodiments disclose three separate debriding apparatuses: (i) an abrasive region at a distal end of the instrument - e.g. Fig. 2, (ii) a liquid passage for injecting/extracting debriding liquid and solids - e.g. Figs. 3A-5B, and (iii) a deployable debriding tool - e.g. Figs. 5A and 5B. It is to be understood that in some embodiments, only one of these debriding apparatuses may be present; however, in some other embodiments any two may be present, and in some further embodiments, all three may be present. It is to be understood that where a liquid passage is absent, the outside surface of the instrument may be defined by the sleeve 318 rather than by the jacket 340. Furthermore, in another embodiment, the moveable electrodes of the embodiment of Figs. 4A and 4B may be included in the deployable debriding tool embodiment of Figs. 5A and 5B.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the appended claims.

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An electrosurgical instrument, comprising:

   an elongate probe comprising a coaxial cable for conveying radiofrequency (RF) and/or microwave frequency electromagnetic (EM) energy, and a probe tip connected at the distal end of the coaxial cable for receiving the RF and/or microwave energy;
   a gas passage for conveying gas through the elongate probe to the probe tip; and
   a debriding apparatus for debriding biological tissue,
   wherein the coaxial cable comprises an inner conductor, an outer conductor and a dielectric material separating the inner conductor from the outer conductor,
   wherein the probe tip comprises a first electrode connected to the inner conductor of the coaxial cable and a second electrode connected to the outer conductor of the coaxial cable, and
   wherein the first electrode and the second electrode are arranged to produce an electric field from the received RF and/or microwave frequency EM energy across a flow path of gas received from the gas passage to produce a thermal or non-thermal plasma in an area outward from the probe tip.

2. An electrosurgical instrument according to claim 1, wherein the first electrode and the second electrode are movable relative to each other into a second configuration in which the first electrode extends distally beyond the second electrode to form a radiating structure for emitting a microwave EM field outwardly from the probe tip.

3. An electrosurgical instrument according to any preceding claim, wherein at least one of the following applies:

   a) the debriding apparatus comprises an abrasive region on a distal outer surface of the probe tip for debriding biological tissue at the probe tip;
   b) the debriding apparatus comprises a debriding tool, and wherein the probe tip comprises a holder for receiving the debriding tool, the debriding tool being moveable between a stowed position, in which the debriding tool is enclosed within the holder, and a deployed position, in which the debriding tool protrudes into the area outward from the probe tip for debriding biological tissue.

4. An electrosurgical instrument according to any preceding claim, wherein the elongate probe comprises a sleeve surrounding the coaxial cable, the gas passage being a space between an inside surface of the sleeve and an outside surface of the coaxial cable, and wherein at least one of the following applies:

   a) the second electrode is formed on the distal end of the sleeve, and the sleeve is retractable relative to the coaxial cable;
   b) the sleeve comprises a rotatable braided cable to permit adjustment of an orientation of the debriding apparatus.

5. An electrosurgical instrument according to any preceding claim, wherein the debriding apparatus comprises a liquid passage for conveying liquid through the elongate probe and into or out of the area outward of the probe tip for debriding biological tissue.

6. An electrosurgical instrument according to claim 5, when dependent on claim 4, wherein either:

   a) the elongate probe comprises a jacket surrounding the sleeve, the liquid passage being a space between the inside surface of the jacket and an outside surface of the sleeve; or
   b) the space between the inside surface of the sleeve and the outside surface of the coaxial cable is partitioned into the gas passage and the liquid passage.

7. An electrosurgical instrument according to claim 5 or 6, wherein the liquid passage is partitioned into a first channel for conveying liquid through the elongate probe into the area outward of the probe tip, and a second channel for conveying liquid through the elongate probe out of the area outward of the probe tip.

8. An electrosurgical instrument according to any preceding claim, wherein at least one of the following applies:

a) the first electrode is a radiating microwave monopole antenna structure coupled to receive RF and/or microwave EM energy from the coaxial cable;
b) the first electrode and the second electrode form active and return electrodes for an RF signal conveyed by the coaxial cable;
c) the outer electrode of the coaxial cable is connected to the second electrode by a gas permeable conductive structure that is slidable relative to the second electrode or outer electrode of the coaxial cable and permits gas to flow through it;
d) the probe is insertable through the instrument channel of a scoping device.

9. Electrosurgical apparatus comprising:

a radiofrequency (RF) signal generator for generating RF electromagnetic (EM) radiation having a first frequency;
a microwave signal generator for generating microwave EM radiation having a second frequency that is higher than the first frequency;
an electrosurgical instrument according to any preceding claim connected to receive the RF EM radiation and the microwave EM radiation;
a feed structure for conveying the RF EM radiation and the microwave EM radiation to the elongate probe, the feed structure comprising an RF channel for connecting the elongate probe to the RF signal generator, and a microwave channel for connecting the elongate probe to the microwave signal generator,
a gas feed connected to supply gas to the electrosurgical instrument,
wherein the apparatus is operable to debride biological tissue in the area outward from the probe tip, and
wherein the apparatus is operable to deliver a thermal or non-thermal plasma in the area outward from the probe tip.

10. Electrosurgical apparatus according to claim 9, when dependent on claim 5, comprising:

a liquid feed connected to supply liquid to, or extract liquid from, the electrosurgical instrument,
wherein the apparatus is operable to supply liquid to, or extract liquid from, the area outward from the probe tip for debriding biological tissue.

11. Electrosurgical apparatus according to claim 10, when dependent on claim 7, wherein the liquid feed comprises:

an injection device for supplying liquid to the electrosurgical instrument via the first channel, and
a suction device for extracting liquid from the electrosurgical instrument via the second channel.

12. Electrosurgical apparatus according to any one of claims 9 to 11, wherein the first electrode and the second electrode are movable relative to each other into a second configuration in which the first electrode extends distally beyond the second electrode to form a radiating structure for emitting a microwave EM field outwardly from the probe tip, and
wherein the apparatus is operable to emit a non-ionising electric field outwardly from the probe tip when the first electrode and the second electrode are in the second configuration without gas supplied to thereto.

13. Electrosurgical apparatus according to any one of claims 9 to 12 comprising:

a microwave signal detector for sampling forward and reflected power on the microwave channel and generating therefrom a microwave detection signal indicative of the microwave power delivered by the probe; and
a controller in communication with the microwave signal detector to receive the microwave detection signal,
wherein the controller is operable to select a first energy delivery profile for the microwave EM energy, the first energy delivery profile for the microwave EM energy being for sterilization of tissue,
wherein the controller comprises a digital microprocessor programmed to output a first microwave control signal for the microwave signal generator, the first microwave control signal being for setting the first energy delivery profile for the microwave EM energy,
wherein the controller is arranged to determine a state for the first microwave control signal based on the received microwave detection signal,
wherein the controller is operable to select a second energy delivery profile for the microwave EM energy, the second energy delivery profile for the microwave EM energy being for resurfacing of tissue, and

wherein the digital microprocessor is programmed to output a second microwave control signal for the microwave signal generator, the second microwave control signal being for setting the second energy delivery profile for the microwave EM energy.

14. Electrosurgical apparatus according to claim 13 including a movement mechanism for causing relative movement between the first electrode and second electrode, wherein the controller is arranged to communicate a control signal to the movement mechanism based on the received microwave detection signal,

wherein the movement mechanism comprises any one of a linear motor, a stepper motor, a piezoelectric actuator, and a magnetostrictive actuator.

15. Electrosurgical apparatus according to claim 13 or 14, when dependent on claim 3, comprising a deployment mechanism for causing relative movement between the debriding tool and the holder, wherein the controller is arranged to communicate a debriding signal to the deployment mechanism for moving the debriding tool between the stowed and deployed positions.

**Patentansprüche**

1. Elektrochirurgisches Instrument, das Folgendes umfasst:

eine längliche Sonde, die ein Koaxialkabel umfasst, zum Übertragen von elektromagnetischer (EM-) Hochfrequenz- (HF-) und/oder Mikrowellenfrequenzenergie, und eine Sondenspitze, die am distalen Ende des Koaxialkabels angeschlossen ist, um die HF- und/oder Mikrowellenenergie zu empfangen;

einen Gasdurchlass zum Leiten von Gas durch die längliche Sonde zur Sondenspitze;

und eine Debridierungsvorrichtung zum Debridieren von biologischem Gewebe,

wobei das Koaxialkabel einen Innenleiter, einen Außenleiter und ein dielektrisches Material umfasst, das den Innenleiter vom Außenleiter trennt,

wobei die Sondenspitze eine erste Elektrode, die mit dem Innenleiter des Koaxialkabels verbunden ist, und eine zweite Elektrode, die mit dem Außenleiter des Koaxialkabels verbunden ist, umfasst und

wobei die erste Elektrode und die zweite Elektrode angeordnet sind, um aus der empfangenen EM-HF- und/oder Mikrowellenfrequenzenergie ein elektrisches Feld quer zu einem Strömungspfad für Gas, das vom Gasdurchlass empfangen wurde, zu erzeugen, um ein thermisches oder nichtthermisches Plasma in einem Bereich außerhalb der Sondenspitze zu erzeugen.

2. Elektrochirurgisches Instrument nach Anspruch 1, wobei die erste Elektrode und die zweite Elektrode relativ zueinander in eine zweite Konfiguration bewegbar sind, in der sich die erste Elektrode distal über die zweite Elektrode hinaus erstreckt, um eine strahlende Struktur zum Emittieren eines Mikrowellen-EM-Felds aus der Sondenspitze heraus auszubilden.

3. Elektrochirurgisches Instrument nach einem der vorangegangenen Ansprüche, wobei zumindest eine der folgenden Aussagen zutrifft:

a) die Debridierungsvorrichtung umfasst eine abrasive Region auf einer distalen Außenfläche der Sondenspitze zum Debridieren von biologischem Gewebe an der Sondenspitze;
b) die Debridierungsvorrichtung umfasst ein Debridierungswerkzeug, und wobei die Sondenspitze eine Halterung zum Aufnehmen des Debridierungswerkzeugs umfasst, wobei das Debridierungswerkzeug zwischen einer verstauten Position, in der das Debridierungswerkzeug innerhalb der Halterung eingehaust ist, und einer ausgefahrenen Position, in der das Debridierungswerkzeug in den Bereich außerhalb der Sondenspitze wegsteht, um biologisches Gewebe zu debridieren, bewegbar ist.

4. Elektrochirurgisches Instrument nach einem der vorangegangenen Ansprüche, wobei die längliche Sonde eine Hülse, die das Koaxialkabel umgibt, umfasst, wobei der Gasdurchlass ein Raum zwischen einer Innenfläche der Hülse und einer Außenfläche des Koaxialkabels ist und wobei zumindest eine von folgenden Aussagen zutrifft:

a) die zweite Elektrode ist auf dem distalen Ende der Hülse ausgebildet, und die Hülse ist relativ zum Koaxialkabel zurückziehbar;
b) die Hülse umfasst ein drehbares geflochtenes Kabel, um ein Anpassen der Ausrichtung der Debridierungsvorrichtung zu ermöglichen.

5. Elektrochirurgisches Instrument nach einem der vorangegangenen Ansprüche, wobei die Debridierungsvorrichtung einen Flüssigkeitsdurchlass zum Leiten von Flüssigkeit durch die längliche Sonde und in den Bereich/aus dem Bereich außerhalb der Sondenspitze umfasst, um biologisches Gewebe zu debridieren.

6. Elektrochirurgisches Instrument nach Anspruch 5, wenn er von Anspruch 4 abhängig ist, wobei entweder:

     a) die längliche Sonde eine die Hülse umgebende Ummantelung umfasst, wobei der Flüssigkeitsdurchlass ein Raum zwischen der Innenfläche der Ummantelung und einer Außenfläche der Hülse ist; oder
     b) der Raum zwischen der Innenfläche der Hülse und der Außenfläche des Koaxialkabels in den Gasdurchlass und den Flüssigkeitsdurchlass unterteilt ist.

7. Elektrochirurgisches Instrument nach Anspruch 5 oder 6, wobei der Flüssigkeitsdurchlass in einen ersten Kanal zum Leiten von Flüssigkeit durch die längliche Sonde in den Bereich außerhalb der Sondenspitze und einen zweiten Kanal zum Leiten von Flüssigkeit durch die längliche Sonde in den Bereich außerhalb der Sondenspitze umfasst.

8. Elektrochirurgisches Instrument nach einem der vorangegangenen Ansprüche, wobei zumindest eine der folgenden Aussagen zutrifft:

     a) die erste Elektrode ist eine strahlende Mikrowellenmonopolantennenstruktur, die gekoppelt ist, um HF- und/oder Mikrowellen-EM-Energie vom Koaxialkabel zu empfangen;
     b) die erste Elektrode und die zweite Elektrode bilden eine aktive und eine Rückelektrode für ein vom Koaxialkabel übertragenes HF-Signal;
     c) die Außenelektrode des Koaxialkabels ist mit der zweiten Elektrode durch eine gasdurchlässige, leitfähige Struktur verbunden, die relativ zur zweiten Elektrode oder zur Außenelektrode des Koaxialkabels verschiebbar ist und das Strömen von Gas dadurch hindurch ermöglicht;
     d) die Sonde ist durch den Instrumentenkanal einer Sondierungsvorrichtung einbringbar.

9. Elektrochirurgische Vorrichtung, die Folgendes umfasst:

     einen Hochfrequenz-(HF-)Signalgenerator zum Erzeugen von elektromagnetischer (EM-) HF-Strahlung mit einer ersten Frequenz;
     einen Mikrowellensignalgenerator zum Erzeugen von Mikrowellen-EM-Strahlung mit einer zweiten Frequenz, die höher ist als die erste Frequenz;
     ein elektrochirurgisches Instrument nach einem der vorangegangenen Ansprüche, das angeschlossen ist, um die HF-EM-Strahlung und die Mikrowellen-EM-Strahlung zu empfangen;
     eine Zufuhrstruktur zum Übertragen der HF-EM-Strahlung und der Mikrowellen-EM-Strahlung an die längliche Sonde, wobei die Zufuhrstruktur einen HF-Kanal zum Verbinden der länglichen Sonde mit dem HF-Signalgenerator und einen Mikrowellenkanal zum Verbinden der länglichen Sonde mit dem Mikrowellensignalgenerator umfasst,
     eine Gaszufuhrleitung, die angeschlossen ist, um das elektrochirurgische Instrument mit Gas zu versorgen,
     wobei die Vorrichtung bedienbar ist, um biologisches Gewebe im Bereich außerhalb der Sondenspitze zu debridieren, und
     wobei die Vorrichtung bedienbar ist, um ein thermisches oder nichtthermisches Plasma im Bereich außerhalb der Sondenspitze zuzuführen.

10. Elektrochirurgische Vorrichtung nach Anspruch 9, wenn er von Anspruch 5 abhängig ist, die Folgendes umfasst:

     eine Flüssigkeitszufuhrleitung, die angeschlossen ist, um das elektrochirurgische Instrument mit Flüssigkeit zu versorgen oder Flüssigkeit daraus zu extrahieren,
     wobei die Vorrichtung bedienbar ist, um den Bereich außerhalb der Sondenspitze mit Flüssigkeit zu versorgen oder Flüssigkeit daraus zu extrahieren, um biologisches Gewebe zu debridieren.

11. Elektrochirurgische Vorrichtung nach Anspruch 10, wenn er von Anspruch 7 abhängig ist, wobei die Flüssigkeitszufuhrleitung Folgendes umfasst:

     eine Injektionsvorrichtung zum Versorgen des elektrochirurgischen Instruments mit Flüssigkeit über den ersten Kanal und
     eine Ansaugvorrichtung zum Extrahieren von Flüssigkeit aus dem elektrochirurgischen Instrument über den zweiten Kanal.

12. Elektrochirurgische Vorrichtung nach einem der Ansprüche 9 bis 11,

     wobei die erste Elektrode und die zweite Elektrode relativ zueinander in eine zweite Konfiguration bewegbar sind, in der sich die erste Elektrode distal über die zweite Elektrode hinaus erstreckt, um eine strahlende Struktur zum Emittieren eines Mikrowellen-EM-Felds aus der Sondenspitze heraus auszubilden, und
     wobei die Vorrichtung bedienbar ist, um ein nichtionisierendes elektrisches Feld aus der Sondenspitze heraus zu emittieren, wenn die erste Elektrode und die zweite Elektrode sich in der zweiten Konfiguration befinden, ohne dass sie mit Gas versorgt wird.

13. Elektrochirurgische Vorrichtung nach einem der Ansprüche 9 bis 12, die Folgendes umfasst:

einen Mikrowellensignaldetektor zum Abtasten von Vorwärts- und reflektierter Leistung im Mikrowellenkanal und Erzeugen eines Mikrowellendetektionssignals daraus, das die durch die Sonde zugeführte Mikrowellenleistung angibt; und
eine Steuerung, die mit dem Mikrowellensignaldetektor in Kommunikation steht, um das Mikrowellendetektionssignal zu empfangen,
wobei die Steuerung betreibbar ist, um ein erstes Energiezufuhrprofil für die Mikrowellen-EM-Energie auszuwählen, wobei das erste Energiezufuhrprofil für die Mikrowelle-EM-Energie der Sterilisierung von Gewebe dient,
wobei die Steuerung einen digitalen Mikroprozessor umfasst, der programmiert ist, um ein erstes Mikrowellensteuersignal für den Mikrowellensignalgenerator auszugeben, wobei das erste Mikrowellensteuersignal dem Einstellen des ersten Energiezufuhrprofils für die Mikrowellen-EM-Energie dient,
wobei die Steuerung angeordnet ist, um einen Zustand für das erste Mikrowellensteuersignal basierend auf dem empfangenen Mikrowellendetektionssignal zu bestimmen,
wobei die Steuerung betreibbar ist, um ein zweites Energiezufuhrprofil für die Mikrowellen-EM-Energie auszuwählen, wobei das zweite Energiezufuhrprofil für die Mikrowellen-EM-Energie der Oberflächenerneuerung von Gewebe dient, und
wobei der digitale Mikroprozessor programmiert ist, um ein zweites Mikrowellensteuersignal für den Mikrowellensignalgenerator auszugeben, wobei das zweite Mikrowellensteuersignal dem Einstellen des zweiten Energiezufuhrprofils für die Mikrowellen-EM-Energie dient.

14. Elektrochirurgische Vorrichtung nach Anspruch 13, umfassend einen Bewegungsmechanismus zum Bewirken einer Relativbewegung zwischen der ersten Elektrode und der zweiten Elektrode, wobei die Steuerung angeordnet ist, um dem Bewegungsmechanismus ein Steuersignal basierend auf dem empfangenen Mikrowellendetektionssignal zu senden, wobei der Bewegungsmechanismus eines aus einem Linearmotor, einem Stufenmotor, einem piezoelektrischen Aktor und einem magnetostriktiven Aktuator umfasst.

15. Elektrochirurgische Vorrichtung nach Anspruch 13 oder 14, wenn sie von Anspruch 3 abhängig sind, umfassend einen Ausfahrmechanismus zum Bewirken einer Relativbewegung zwischen dem Debridierungswerkzeug und der Halterung, wobei die Steuerung angeordnet ist, um ein Debridierungssignal an den Ausfahrmechanismus zu senden, um das Debridierungswerkzeug zwischen der verstauten und der ausgefahrenen Position zu bewegen.

## Revendications

1. Instrument électrochirurgical, comprenant :

une sonde allongée comprenant un câble coaxial pour transporter une énergie électromagnétique (EM) de radiofréquence (RF) et/ou de fréquence micro-onde, et une pointe de sonde reliée à l'extrémité distale du câble coaxial pour recevoir l'énergie RF et/ou micro-onde ;
un passage de gaz pour transporter le gaz à travers la sonde allongée vers la pointe de sonde ; et
un appareil de débridage pour débrider le tissu biologique,
dans lequel le câble coaxial comprend un conducteur interne, un conducteur externe et un matériau diélectrique séparant le conducteur interne du conducteur externe,
dans lequel la pointe de sonde comprend une première électrode reliée au conducteur interne du câble coaxial et une seconde électrode reliée au conducteur externe du câble coaxial, et
dans lequel la première électrode et la seconde électrode sont agencées pour produire un champ électrique à partir de l'énergie EM de RF et/ou de fréquence micro-onde reçue à travers un trajet d'écoulement de gaz reçu depuis le passage de gaz pour produire un plasma thermique ou non thermique dans une zone à l'extérieur de la pointe de sonde.

2. Instrument électrochirurgical selon la revendication 1, dans lequel la première électrode et la seconde électrode sont mobiles l'une par rapport à l'autre dans une seconde configuration dans laquelle la première électrode s'étend de manière distale au-delà de la seconde électrode pour former une structure rayonnante pour émettre un champ EM micro-onde à l'extérieur de la pointe de sonde.

3. Instrument électrochirurgical selon une quelconque revendication précédente, dans lequel au moins l'un des points suivants s'applique :

a) l'appareil de débridage comprend une région abrasive sur une surface externe distale de la pointe de sonde pour débrider le tissu biologique au niveau de la pointe de sonde ;

b) l'appareil de débridage comprend un outil de débridage, et dans lequel la pointe de sonde comprend un support pour recevoir l'outil de débridage, l'outil de débridage étant mobile entre une position repliée, dans laquelle l'outil de débridage est enfermé à l'intérieur du support, et une position déployée, dans laquelle l'outil de débridage fait saillie dans la zone à l'extérieur de la pointe de sonde pour débrider le tissu biologique.

4. Instrument électrochirurgical selon une quelconque revendication précédente, dans lequel la sonde allongée comprend un manchon entourant le câble coaxial, le passage de gaz étant un espace entre une surface intérieure du manchon et une surface extérieure du câble coaxial, et dans lequel au moins l'un des points suivants s'applique :

a) la seconde électrode est formée sur l'extrémité distale du manchon, et le manchon est rétractable par rapport au câble coaxial ;
b) le manchon comprend un câble tressé rotatif pour permettre l'ajustement d'une orientation de l'appareil de débridage.

5. Instrument électrochirurgical selon une quelconque revendication précédente, dans lequel l'appareil de débridage comprend un passage de liquide pour transporter du liquide à travers la sonde allongée et dans ou hors de la zone à l'extérieur de la pointe de sonde pour débrider le tissu biologique.

6. Instrument électrochirurgical selon la revendication 5, lorsqu'elle dépend de la revendication 4, dans lequel soit :

a) la sonde allongée comprend une gaine entourant le manchon, le passage de liquide étant un espace entre la surface intérieure de la gaine et une surface extérieure du manchon ; ou
b) l'espace entre la surface intérieure du manchon et la surface extérieure du câble coaxial est divisé en le passage de gaz et le passage de liquide.

7. Instrument électrochirurgical selon la revendication 5 ou 6, dans lequel le passage de liquide est divisé en un premier canal pour transporter du liquide à travers la sonde allongée dans la zone à l'extérieur de la pointe de sonde, et un second canal pour transporter du liquide à travers la sonde allongée hors de la zone à l'extérieur de la pointe de sonde.

8. Instrument électrochirurgical selon une quelconque revendication précédente, dans lequel au moins l'un des points suivants s'applique :

a) la première électrode est une structure d'antenne unipolaire micro-onde rayonnante couplée pour recevoir de l'énergie EM RF et/ou micro-onde du câble coaxial ;
b) la première électrode et la seconde électrode forment des électrodes active et de retour pour un signal RF transporté par le câble coaxial ;
c) l'électrode externe du câble coaxial est reliée à la seconde électrode par une structure conductrice perméable au gaz qui est coulissante par rapport à la seconde électrode ou à l'électrode externe du câble coaxial et permet au gaz de s'écouler à travers elle ;
d) la sonde est insérable à travers le canal d'instrument d'un dispositif d'endoscopie.

9. Appareil électrochirurgical comprenant :

un générateur de signal radiofréquence (RF) pour générer un rayonnement électromagnétique (EM) RF ayant une première fréquence ;
un générateur de signal micro-onde pour générer un rayonnement EM micro-onde ayant une seconde fréquence qui est supérieure à la première fréquence ;
un instrument électrochirurgical selon une quelconque revendication précédente relié pour recevoir le rayonnement EM RF et le rayonnement EM micro-onde ;
une structure d'alimentation pour transporter le rayonnement EM RF et le rayonnement EM micro-onde vers la sonde allongée, la structure d'alimentation comprenant un canal RF pour relier la sonde allongée au générateur de signal RF, et un canal micro-onde pour relier la sonde allongée au générateur de signal micro-onde,
une alimentation en gaz reliée pour fournir du gaz à l'instrument électrochirurgical,
dans lequel l'appareil sert à débrider le tissu biologique dans la zone à l'extérieur de la pointe de sonde, et
dans lequel l'appareil sert à fournir un plasma thermique ou non thermique dans la zone à l'extérieur de la pointe de sonde.

10. Appareil électrochirurgical selon la revendication 9, lorsqu'elle dépend de la revendication 5, comprenant :

une alimentation en liquide reliée pour fournir du liquide à l'instrument chirurgical ou pour extraire du liquide de celui-ci,
dans lequel l'appareil sert à fournir du liquide à la zone à l'extérieur de la pointe de sonde ou à extraire du liquide de celle-ci pour débrider le tissu biologique.

11. Appareil électrochirurgical selon la revendication 10, lorsqu'elle dépend de la revendication 7, dans lequel l'alimentation en liquide comprend :

un dispositif d'injection pour fournir du liquide à l'instrument électrochirurgical via le premier canal, et

un dispositif d'aspiration pour extraire du liquide de l'instrument électrochirurgical via le second canal.

12. Appareil électrochirurgical selon l'une quelconque des revendications 9 à 11, dans lequel la première électrode et la seconde électrode sont mobiles l'une par rapport à l'autre dans une seconde configuration dans laquelle la première électrode s'étend de manière distale au-delà de la seconde électrode pour former une structure rayonnante destinée à émettre un champ EM micro-onde à l'extérieur de la pointe de sonde, et

dans lequel l'appareil sert à émettre un champ électrique non ionisant à l'extérieur de la pointe de sonde lorsque la première électrode et la seconde électrode sont dans la seconde configuration sans que du gaz lui soit fourni.

13. Appareil électrochirurgical selon l'une quelconque des revendications 9 à 12 comprenant :

un détecteur de signal micro-onde pour échantillonner une puissance directe et réfléchie sur le canal micro-onde et générer à partir de celle-ci un signal de détection de micro-onde indicateur de la puissance micro-onde fournie par la sonde ; et

un dispositif de commande en communication avec le détecteur de signal micro-onde pour recevoir le signal de détection de micro-onde,

dans lequel le dispositif de commande sert à sélectionner un premier profil de fourniture d'énergie pour l'énergie EM micro-onde, le premier profil de fourniture d'énergie pour l'énergie EM micro-onde étant destiné à la stérilisation de tissu,

dans lequel le dispositif de commande comprend un microprocesseur numérique programmé pour délivrer en sortie un premier signal de commande de micro-onde pour le générateur de signal micro-onde, le premier signal de commande de micro-onde étant destiné à définir le premier profil de fourniture d'énergie pour l'énergie EM micro-onde,

dans lequel le dispositif de commande est agencé pour déterminer un état pour le premier signal de commande de micro-onde sur la base du signal de détection de micro-onde reçu,

dans lequel le dispositif de commande sert à sélectionner un second profil de fourniture d'énergie pour l'énergie EM micro-onde, le second profil de fourniture d'énergie pour l'énergie EM micro-onde étant destiné au resurfaçage de tissu, et

dans lequel le microprocesseur numérique est programmé pour délivrer en sortie un second signal de commande de micro-onde pour le générateur de signal micro-onde, le second signal de commande de micro-onde étant destiné à définir le second profil de fourniture d'énergie pour l'énergie EM micro-onde.

14. Appareil électrochirurgical selon la revendication 13 incluant un mécanisme de déplacement pour provoquer un déplacement relatif entre la première électrode et la seconde électrode, dans lequel le dispositif de commande est agencé pour communiquer un signal de commande au mécanisme de déplacement sur la base du signal de détection de micro-onde reçu,

dans lequel le mécanisme de déplacement comprend l'un quelconque d'un moteur linéaire, d'un moteur pas-à-pas, d'un actionneur piézoélectrique ou d'un actionneur magnétostrictif.

15. Appareil électrochirurgical selon la revendication 13 ou 14, lorsqu'elles dépendent de la revendication 3, comprenant un mécanisme de déploiement pour provoquer un déplacement relatif entre l'outil de débridage et le support, dans lequel le dispositif de commande est agencé pour communiquer un signal de débridage au mécanisme de déploiement pour déplacer l'outil de débridage entre les positions pliée et déployée.

FIG. 1 (Prior Art)

EP 4 076 238 B1

FIG. 2

EP 4 076 238 B1

FIG. 3A

FIG. 3B

EP 4 076 238 B1

FIG. 4A

FIG. 4B

FIG. 5A

FIG. 5B

**REFERENCES CITED IN THE DESCRIPTION**

**Patent documents cited in the description**

- WO 2009060213 A
- US 2016113700 A1

- WO 2012076844 A